# EUROPÄISCHE PATENTSCHRIFT

(11) **EP 1 999 226 B1**
(45) Veröffentlichungstag und Bekanntmachung des Hinweises auf die Patenterteilung: **12.08.2015**
(21) Anmeldenummer: 07722982.1
(22) Anmeldetag: 28.02.2007
(51) Int. Cl.: C09K 11/06, H05B 33/14, C07C 15/28, H01L 51/00, C07C 43/275, C07C 211/61, C07C 211/58, C07C 13/567, C07C 15/52, C07C 211/56, H01L 51/50, C07C 211/54

(54) **NEUE MATERIALIEN FÜR ORGANISCHE ELEKTROLUMINESZENZVORRICHTUNGEN**
NEW MATERIALS FOR ORGANIC ELECTROLUMINESCENT DEVICES
NOUVEAUX MATERIAUX POUR DISPOSIFS ELECTROLUMINESCENTS ORGANIQUES

(30) Priorität: 24.03.2006 DE 102006013802
(43) Veröffentlichungstag der Anmeldung: 10.12.2008
(73) Patentinhaber: Merck Patent GmbH, 64293 Darmstadt (DE)
(72) Erfinder: STOESSEL, Philipp, 60487 Frankfurt am Main (DE); HEIL, Holger, 64295 Darmstadt (DE); PARHAM, Amir, Hossain, 65929 Frankfurt am Main (DE); VESTWEBER, Horst, 34630 Gilersberg-Winterscheid (DE)
(86) Internationale Anmeldenummer: PCT/EP2007/001732
(87) Internationale Veröffentlichungsnummer: WO 2007/110129

(56) Entgegenhaltungen:
- EP-A- 1 734 038
- WO-A-03/060956
- WO-A-03/095445

## Beschreibung

Die vorliegende Erfindung betrifft neue Anthracenderivate, deren Verwendung in organischen Elektrolumineszenzvorrichtungen und organische Elektrolumineszenzvorrichtungen enthaltend diese Verbindungen.

Organische Halbleiter werden als funktionelle Materialien in einer Reihe verschiedenartiger Anwendungen, die im weitesten Sinn der Elektronikindustrie zugerechnet werden können, verwendet. Der allgemeine Aufbau organischer Elektrolumineszenzvorrichtungen, die zur Emission von Licht im sichtbaren Spektralbereich befähigt sind, ist beispielsweise in US 4539507, US 5151629, EP 0676461 und WO 98/27136 beschrieben.

Allerdings zeigen diese Vorrichtungen immer noch erhebliche Probleme, die für die Verwendung in hochwertigen Vollfarbdisplays einer dringenden Verbesserung bedürfen:
1. Effizienz, Farbe und Lebensdauer der organischen Elektrolumineszenzvorrichtungen sind noch nicht ausreichend gut für hochwertige Anwedungen.
2. Die verwendeten Verbindungen weisen häufig keine ausreichend hohe Glasübergangstemperatur auf.
3. Die Redoxstabilität (Stabilität gegenüber Löchern und Elektronen) der bislang verwendeten Verbindungen ist noch nicht ausreichend.
4. Die Ladungsträgermobilität, insbesondere die Elektronenmobilität, ist nicht ausreichend.
5. Die Betriebsspannung sollte, insbesondere für mobile Anwendungen, noch weiter reduziert werden.

Als nächstliegender Stand der Technik kann die Verwendung verschiedener kondensierter Aromaten, insbesondere Anthracen- oder Pyrenderivate, als Host-Materialien vor allem für blau emittierende Elektrolumineszenzvorrichtungen genannt werden. Als Host-Material gemäß dem Stand der Technik ist 9,10-Bis(2-naphthyl)anthracen (US 5935721) bekannt. Weitere Anthracenderivate, die sich als Host-Materialien eignen, sind in WO 01/076323, in WO 01/021729, in WO 04/013073, in WO 04/018588, in WO 03/087023 oder in WO 04/018587 beschrieben. Host-Materialien, basierend auf arylsubstituierten Pyrenen und Chrysenen, werden in WO 04/016575 beschrieben. In WO 03/095445 und in CN 1362464 werden 9,10-Bis(1-naphthyl)anthracen-Derivate für die Verwendung in OLEDs beschrieben.

Es war die Aufgabe der vorliegenden Erfindung, Verbindungen mit verbesserten Eigenschaften, insbesondere verbesserte Hostmaterialien bereitzustellen.

Überraschend wurde gefunden, dass organische Elektrolumineszenzvorrichtungen, welche Anthracenderivate enthalten, die in 9,10-Position mit ortho-substituierten Phenylgruppen substituiert sind und die weiterhin in 2,6-Position mit Arylgruppen substituiert sind, deutliche Verbesserungen gegenüber dem Stand der Technik aufweisen. Diese Verbindungen und deren Verwendung in OLEDs sind daher der Gegenstand der vorliegenden Erfindung.

Aus WO 03/060956 und WO 05/097756 sind Anthracenderivate bekannt, die in 9,10-Position mit ortho-Biphenyl substituiert sind und die weiterhin in 2,6-Position mit Arylgruppen substituiert sind, die Benzimidazol enthalten. Dabei wird der positive Effekt dieser Verbindungen auf die Anwesenheit der Benzimidazolgruppen zurückgeführt.

Gegenstand der Erfindung sind Verbindungen der Formel (1), 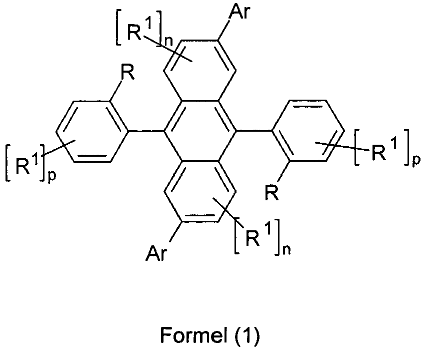 wobei für die verwendeten Symbole und Indizes gilt:
- Ar: ist bei jedem Auftreten gleich oder verschieden eine Arylgruppe mit 6 bis 14 aromatischen Ringatomen, welche mit einem oder mehreren Resten R¹ substituiert sein kann;
- R: steht bei jedem Auftreten gleich oder verschieden für Si(R²)₃, N(Ar¹)₂, C(=O)Ar¹, P(=O)(Ar¹)₂, eine geradkettige Alkyl- oder Alkoxygruppe mit 1 bis 10 C-Atomen oder eine verzweigte oder cyclische Alkyl- oder Alkoxygruppe mit 3 bis 10 C-Atomen, die jeweils mit einem oder mehreren Resten R² substituiert sein kann, wobei eine oder mehrere nicht benachbarte CH₂-Gruppen durch - R²C=CR²- oder -O- ersetzt sein können und wobei ein oder mehrere H-Atome durch F ersetzt sein können, oder für eine Arylgruppe mit 5 bis 16 aromatischen Ringatomen, die mit einem oder mehreren Resten R² substituiert sein kann, oder eine Kombination aus zwei, drei oder vier dieser Systeme; dabei können auch benachbarte Substituenten R und R¹ miteinander ein mono- oder polycyclisches, aliphatisches Ringsystem bilden;
- R¹: steht, gleich oder verschieden bei jedem Auftreten, für Si(R²)₃, F, N(Ar¹)₂, eine geradkettige Alkyl- oder Alkoxygruppe mit 1 bis 6 C-Atomen oder eine verzweigte oder cyclische Alkyl- oder Alkoxygruppe mit 3 bis 10 C-Atomen, wobei jeweils ein oder mehrere CH₂-Gruppen durch -R²C=CR²- oder -O- ersetzt sein können und wobei jeweils ein oder mehrere H-Atome durch F ersetzt sein können, oder eine Arylgruppe mit 5 bis 14 aromatischen Ringatomen, die mit einem oder mehreren Resten R² substituiert sein kann, oder eine Kombination aus zwei oder drei dieser Systeme; dabei können auch zwei oder mehrere Reste R¹ miteinander oder mit einem benachbarten Rest R ein mono- oder polycyclisches, aliphatisches Ringsystem bilden;
- Ar¹: ist bei jedem Auftreten gleich oder verschieden ein aromatisches Ringsystem mit 5 bis 30 aromatischen Ringatomen, welches durch einen oder mehrere Reste nicht-aromatische R¹ substituiert sein kann; dabei können zwei Reste Ar¹ auch durch eine Einfachbindung oder eine Gruppe O, S, N(R²) oder C(R²)₂ miteinander verbunden sein;
- R²: ist bei jedem Auftreten gleich oder verschieden H oder ein Kohlenwasserstoffrest mit 1 bis 20 C-Atomen, welcher aliphatisch oder aromatisch oder eine Kombination aus aliphatisch und aromatisch sein kann und welcher auch durch F substituiert sein kann; dabei können auch zwei oder mehrere Reste R² miteinander ein mono- oder polycyclisches, aliphatisches oder aromatisches Ringsystem bilden;
- n: ist gleich oder verschieden bei jedem Auftreten 0, 1, 2 oder 3;
- p: ist gleich oder verschieden bei jedem Auftreten 0, 1, 2, 3 oder 4.

Unter einer Arylgruppe bzw. einer Heteroarylgruppe im Sinne dieser Erfindung wird eine aromatische Gruppe bzw. heteroaromatische Gruppe mit einem gemeinsamen aromatischen π-Elektronensystem verstanden. Dies kann im Sinne dieser Erfindung ein einfacher Homo- oder Heterocyclus sein, beispielsweise Benzol, Pyridin, etc., oder es kann eine kondensierte Aryl- oder Heteroarylgruppe sein, in der mindestens zwei aromatische oder heteroaromatische Ringe, beispielsweise Benzolringe, miteinander "verschmolzen", d. h. durch Anellierung einander ankondensiert sind, also mindestens eine gemeinsame Kante und dadurch auch ein gemeinsames aromatisches π-Elektronensystem aufweisen. Diese Aryl- oder Heteroarylgruppen können substituiert oder unsubstituiert sein; ebenso können gegebenenfalls vorhandene Substituenten weitere Ringsysteme bilden. So sind beispielsweise Systeme wie Naphthalin, Anthracen, Phenanthren, Pyren, etc. als Arylgruppen und Chinolin, Acridin, Benzothiophen, Carbazol, etc. als Heteroarylgruppen im Sinne dieser Erfindung zu sehen, während beispielsweise Biphenyl, Fluoren, Spirobifluoren, etc. keine Arylgruppen darstellen, da es sich hierbei um separate aromatische Elektronensysteme handelt.

Ein aromatisches Ringsystem im Sinne dieser Erfindung enthält 6 bis 40 C-Atome im Ringsystem. Ein heteroaromatisches Ringsystem im Sinne dieser Erfindung enthält 2 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Diese aromatischen und heteroaromatischen Ringsysteme können substituiert oder unsubstituiert sein; ebenso können gegebenenfalls vorhandene Substituenten weitere Ringsysteme bilden. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine kurze, nicht-aromatische Einheit (bevorzugt weniger als 10 % der von H verschiedenen Atome), wie z. B. ein sp³-hybridisiertes C-, N- oder O-Atom, unterbrochen sein können. So sollen beispielsweise auch Systeme wie 9,9'-Spirobifluoren, Fluoren, 9,9-Diarylfluoren, Triarylamin, Diarylether, etc. als aromatische Ringsysteme im Sinne dieser Erfindung verstanden werden. Dabei kann ein Teil des aromatischen oder heteroaromatischen Ringsystems auch eine kondensierte Gruppe sein.

Unter einer cyclischen Alkylgruppe im Sinne dieser Erfindung werden sowohl monocyclische wie auch bi- und polycyclische Alkylgruppen verstanden.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, besonders bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer C₁ bis C₄₀-Alkoxygruppe werden besonders bevorzugt Methoxy, Trifluormethoxy Ethoxy, n-Propoxy, i-Propoxy, n-Butoxy, i-Butoxy, s-Butoxy, t-Butoxy oder 2-Methylbutoxy verstanden. Unter einem aromatischen oder heteroaromatischen Ringsystem mit 1 bis 30 aromatischen Ringatomen, welches noch jeweils mit den oben genannten Resten R¹ bzw. R² substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Pyren, Chrysen, Perylen, Fluoranthen, Tetracen, Pentacen, Benzpyren, Biphenyl, Biphenylen, Terphenyl, Terphenylen, Fluoren, Spirobifluoren, Truxen, Isotruxen, Dihydrophenanthren, Dihydropyren, Tetrahydropyren, cis- oder trans-Indenofluoren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin, Benzothiadiazol, Triphenylamin, Diphenylnaphthylamin, Dinaphthylphenylamin, Diphenylether, Stilben und Tolan.

Im Folgenden werden bevorzugte Ausführungsformen für Verbindungen der Formel (1) beschrieben.

Bevorzugt steht das Symbol Ar für Phenyl, 2-Pyridyl, 3-Pyridyl, 4-Pyridyl, 1-Naphthyl, 2-Naphthyl, 2-Anthryl, 9-Anthryl, 2-Phenanthrenyl, 3-Phenanthrenyl, 9-Phenanthrenyl, 1-Pyrenyl oder 2-Pyrenyl. Ganz besonders bevorzugt steht das Symbol Ar für Phenyl, 1-Naphthyl, 2-Naphthyl oder 9-Anthryl.

Es sind sowohl Verbindungen gemäß Formel (1) erfindungsgemäß, in denen die beiden Substituenten Ar gleich gewählt sind, wie auch Verbindungen gemäß Formel (1), in denen die Substituenten Ar unterschiedlich sind. In einer bevorzugten Ausführungsform der Erfindung sind die beiden Symbole Ar gleich gewählt. Besonders bevorzugt sind daher die Verbindungen der Formel (2), (3), (4) und (5), 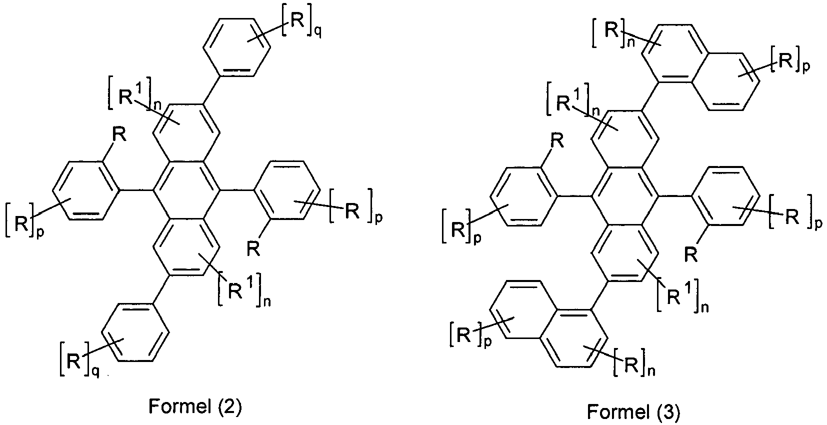 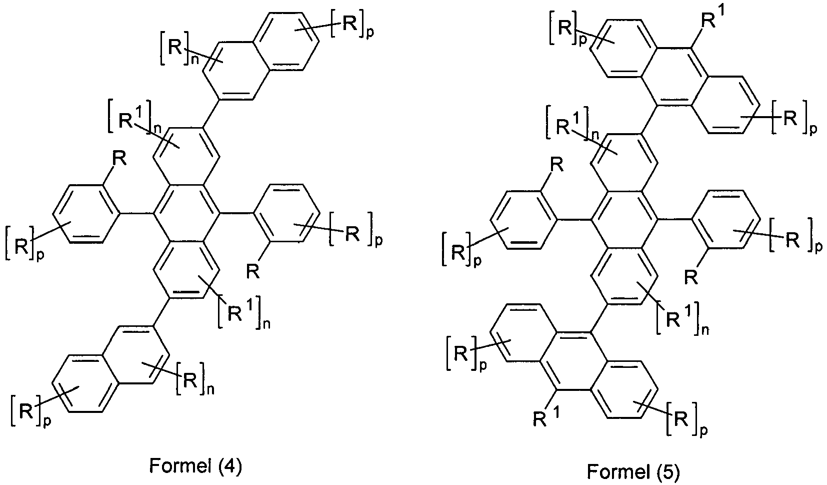 wobei R, R¹, n und p dieselbe Bedeutung haben, wie oben beschrieben, und q für 0, 1, 2, 3, 4 oder 5 steht.

In den Verbindungen gemäß Formel (1) bzw. (2) bis (5) können die Phenylgruppen in 9- und 10-Position am Anthracen gehinderte Rotation um die Anthracen-Phenyl-Bindung aufweisen. Unter gehinderter Rotation im Sinne dieser Erfindung wird eine Rotationsbarriere von mindestens 80 kJ/mol, bevorzugt mindestens 100 kJ/mol, insbesondere mindestens 120 kJ/mol bei Raumtemperatur verstanden. Diese Rotationsbarriere lässt sich experimentell durch temperaturabhängige NMR-Messungen bestimmen. Wenn die Verbindung der Formel (1) bzw. (2) bis (5) Atropisomerie um eine oder um mehrere Bindungen zeigt, so sind jeweils auch die entsprechenden isolierten oder angereicherten Atropisomere Gegenstand der Erfindung. Dies bezieht sich sowohl auf Enantiomere wie auch auf Diastereomere. Gehinderte Rotation um die Anthracen-Phenyl-Bindung wird durch ausreichend große Substituenten R erreicht.

Bevorzugt sind weiterhin Verbindungen der Formel (1) bzw. der Formeln (2) bis (5), in denen das Symbol R für Si(R²)₃, eine geradkettige Alkylgruppe mit 1 bis 4 C-Atomen oder eine verzweigte Alkylgruppe mit 3 bis 5 C-Atomen, die jeweils mit einem oder mehreren Resten R² substituiert sein kann, wobei ein oder mehrere H-Atome durch F ersetzt sein können, oder für eine Arylgruppe mit 6 bis 14 aromatischen Ringatomen, die mit einem oder mehreren Resten R² substituiert sein kann, oder eine Kombination aus zwei oder drei dieser Systeme steht; dabei können auch benachbarte Substituenten R und R¹ miteinander ein mono- oder polycyclisches, aliphatisches Ringsystem bilden.

Es sind sowohl Verbindungen gemäß Formel (1) bzw. (2) bis (5) erfindungsgemäß, in denen die beiden Substituenten R gleich gewählt sind, wie auch Verbindungen gemäß Formel (1) bzw. (2) bis (5), in denen die beiden Substituenten R unterschiedlich sind. Bevorzugt sind die beiden Substituenten R gleich gewählt.

Bevorzugt sind weiterhin Verbindungen gemäß Formel (1) bzw. (2) bis (5), in denen das Symbol R¹ ausgewählt ist aus der Gruppe bestehend aus Si(R²)₃, F, geradkettigen Alkylgruppen mit 1 bis 4 C-Atomen oder verzweigten Alkylgruppen mit 3 bis 5 C-Atomen, wobei jeweils ein oder mehrere H-Atome durch F ersetzt sein können, oder Arylgruppen mit 6 bis 10 aromatischen Ringatomen oder einer Kombination aus zwei dieser Systeme; dabei können auch zwei oder mehrere benachbarte Reste R¹ miteinander oder mit einem benachbarten Rest R ein mono- oder polycyclisches, aliphatisches Ringsystem bilden.

Wenn einer der Reste R und/oder R¹ für eine Gruppe der Formel N(Ar¹)₂ steht, so steht er bevorzugt für eine Gruppe der Formel (6) oder (7), 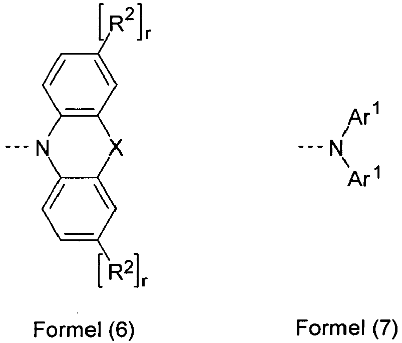 wobei R² die oben aufgeführte Bedeutung hat und weiterhin gilt:
- X: steht für eine Einfachbindung, O, S, N(R²) oder C(R²)₂;
- Ar¹: ist gleich oder verschieden bei jedem Auftreten eine Arylgruppe mit 5 bis 20 aromatischen Ringatomen oder eine Triarylamingruppe mit 15 bis 30 aromatischen Ringatomen, welche jeweils mit einem oder mehreren Resten R¹ substituiert sein kann, bevorzugt eine Arylgruppe mit 6 bis 14 aromatischen Ringatomen oder eine Triarylamingruppe mit 18 bis 26 aromatischen Ringatomen, welche jeweils mit einem oder mehreren nicht-aromatischen Resten R¹ substituiert sein kann, besonders bevorzugt Phenyl, o-Tolyl, p-Tolyl, o-Fluorphenyl, p-Fluorphenyl, 1-Naphthyl, 2-Naphthyl, Triphenylamin oder Naphthyldiphenylamin;
- r: ist bei jedem Auftreten gleich oder verschieden 0 oder 1, bevorzugt 0.

Bevorzugt sind weiterhin Verbindungen gemäß Formel (1) bzw. (2) bis (5), in denen der Index n für 0 oder 1 steht, besonders bevorzugt für 0.

Bevorzugt sind weiterhin Verbindungen gemäß Formel (1) bzw. (2) bis (5), in denen der Index p für 0, 1 oder 2 steht, besonders bevorzugt für 0 oder 1.

Bevorzugt sind weiterhin Verbindungen gemäß Formel (2), in denen der Index q für 0, 1,2 oder 3 steht, besonders bevorzugt für 0, 1 oder 2, ganz besonders bevorzugt für 0 oder 1.

Bevorzugt sind weiterhin Verbindungen gemäß Formel (1), deren Molekulargewicht zwischen 500 und 2000 g/mol liegt, besonders bevorzugt zwischen 600 und 1500 g/mol.

Beispiele für bevorzugte Verbindungen gemäß Formel (1) sind die im Folgenden abgebildeten Verbindungen (1) bis (50).

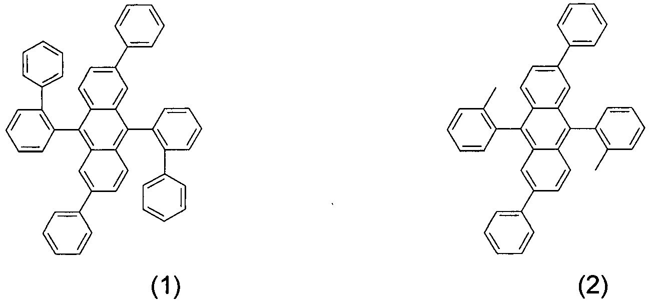

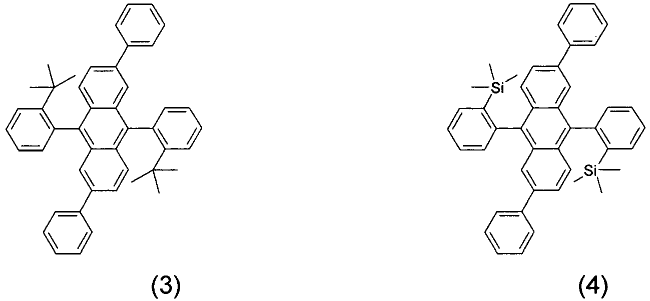

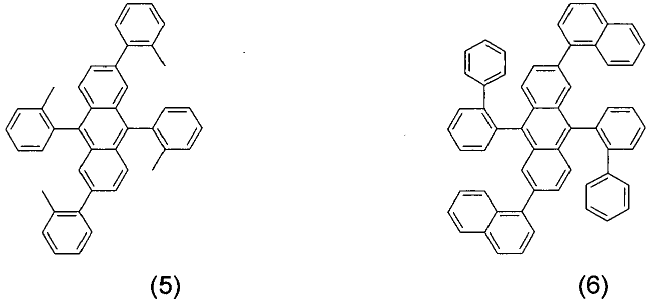

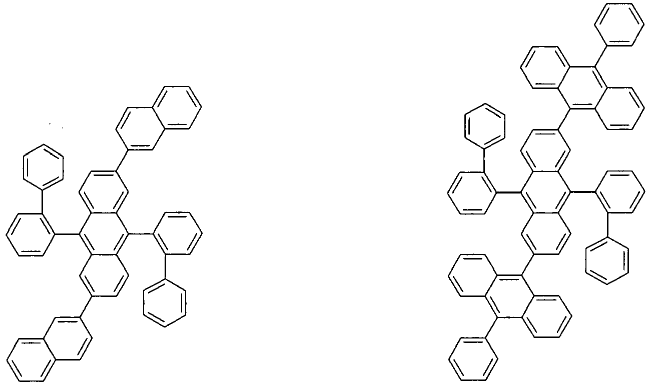

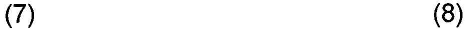

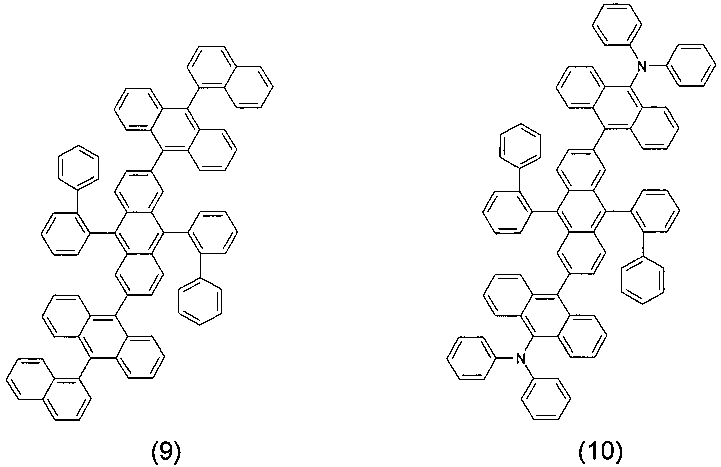

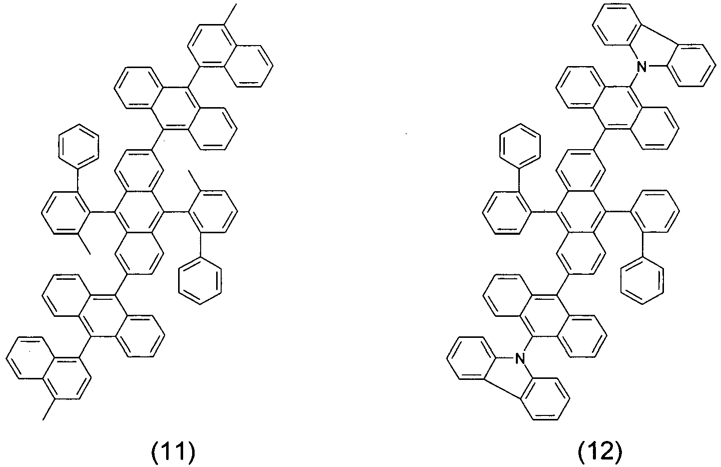

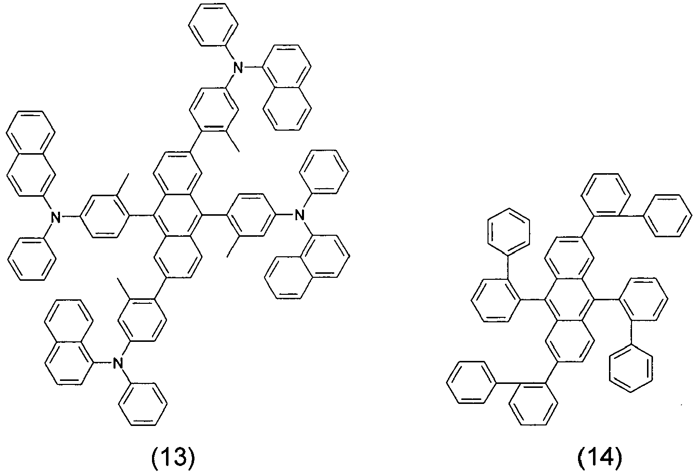

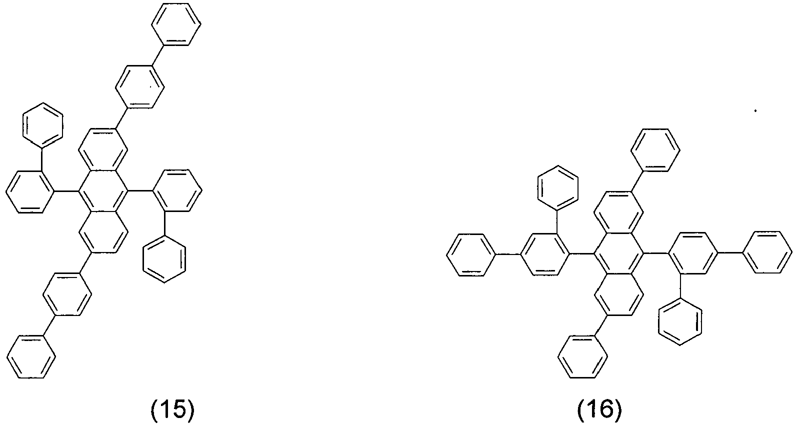

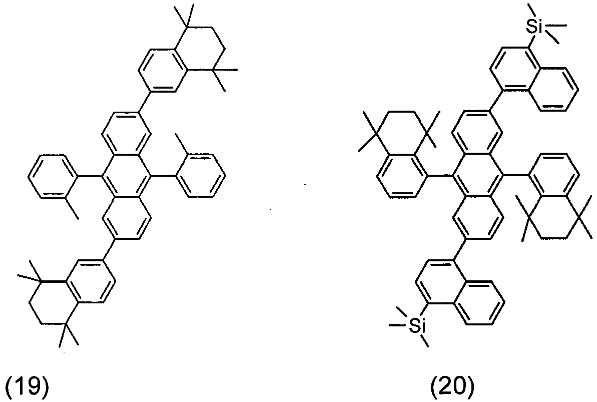

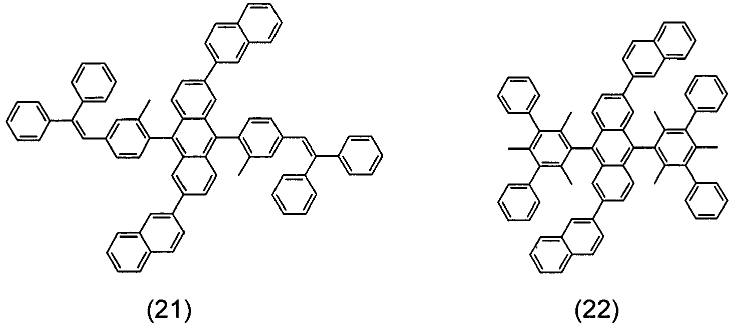

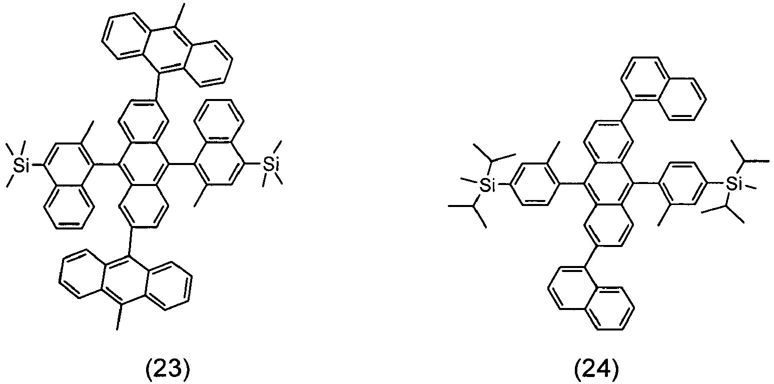

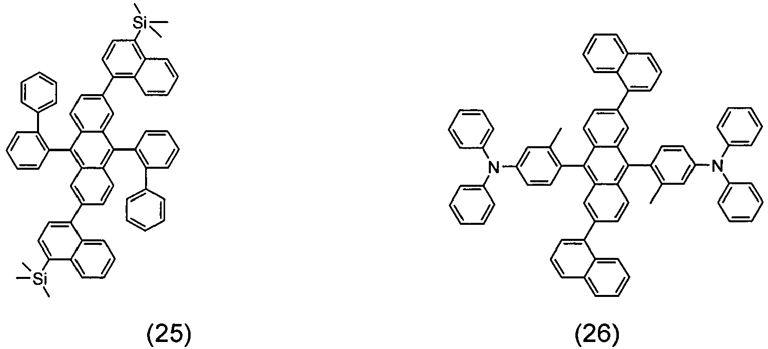

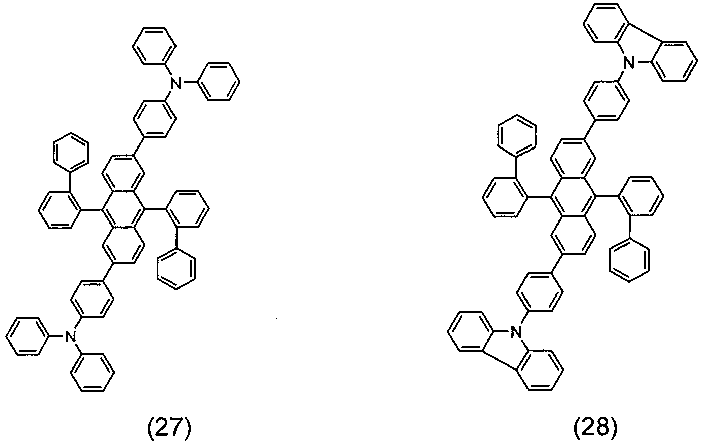

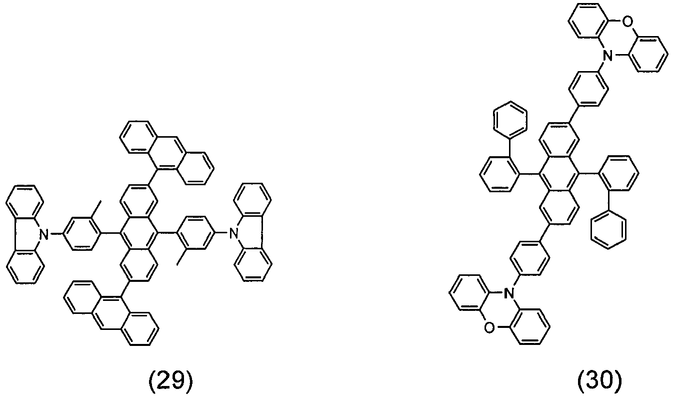

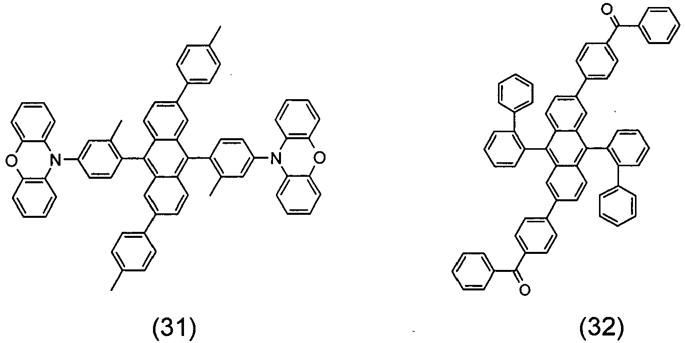

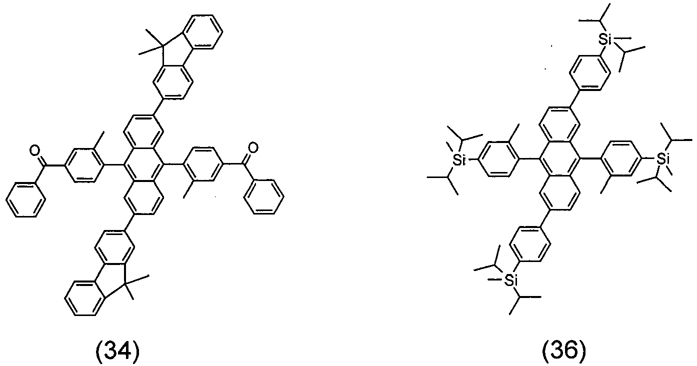

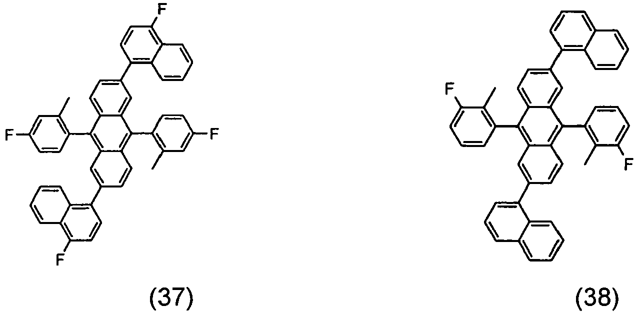

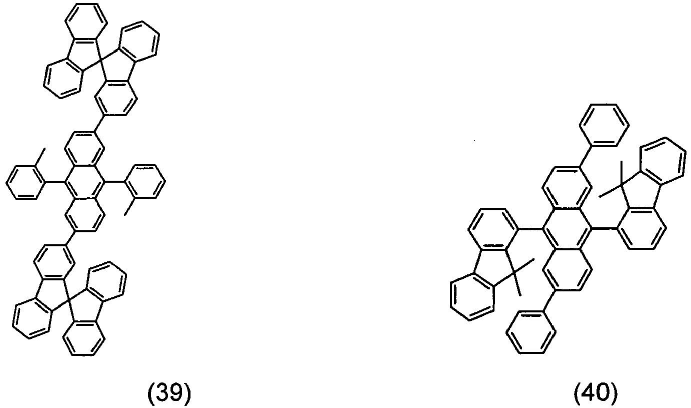

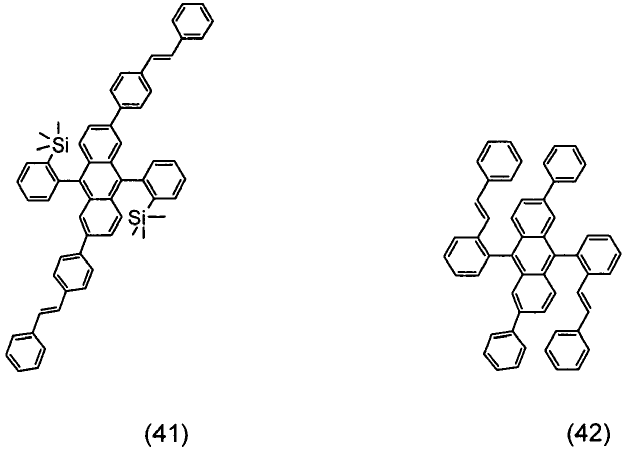

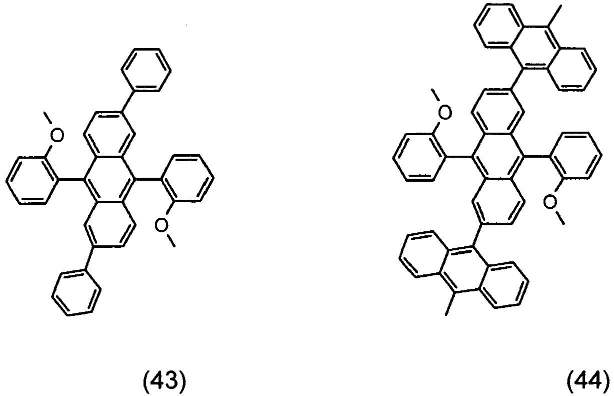

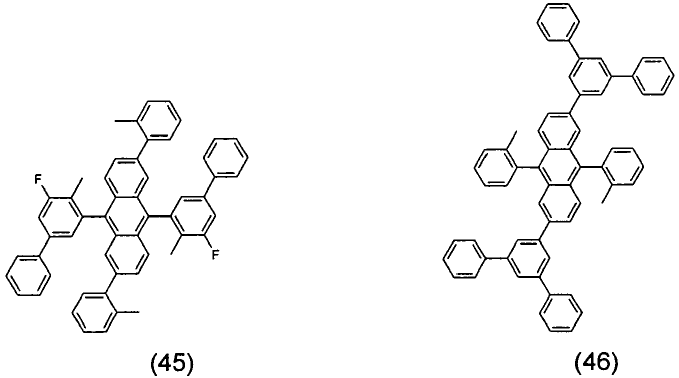

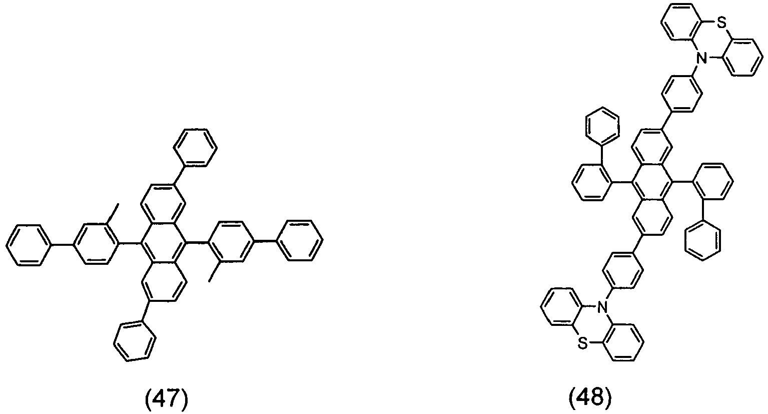

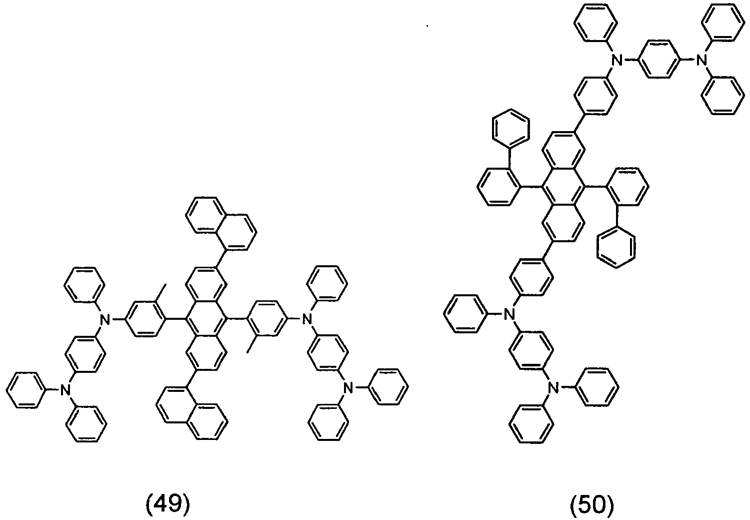

Die folgende Tabelle 1 gibt weitere bevorzugte Strukturen gemäß Formel (1) an. Die in der Tabelle verwendeten Symbole und Indizes beziehen sich auf die im Folgenden abgebildete Formel (8):

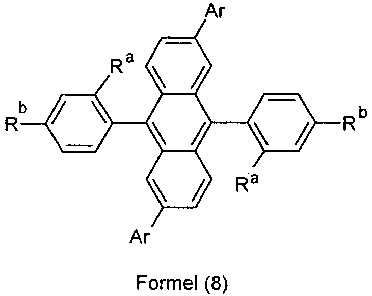

Dabei steht Ar für eine Gruppe der Formel (9), (10) oder (11): 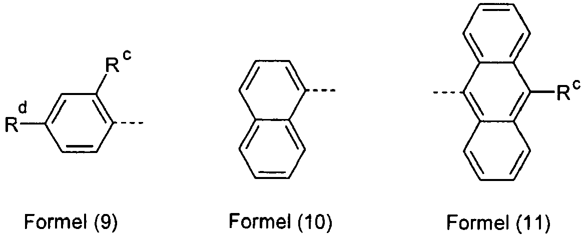 wobei die gestrichelte Bindung die Verknüpfung mit der Anthraceneinheit bedeutet.

Weiterhin steht in Tabelle 1 die Abkürzung N(p-Tol)₂ für eine Bis(paratolyl)aminogruppe.

**Tabelle 1: Bevorzugte Strukturen der Formel (8)**

| Nr. | Ar | Ar | | Ra | Rb |
|---|---|---|---|---|---|
| | | Rc | Rd | | |
| 1 | Phenyl | H | H | Methyl | H |
| 2 | Phenyl | H | H | Methyl | Methyl |
| 3 | Phenyl | H | H | Methyl | tert-Butyl |
| 4 | Phenyl | H | H | Methyl | Si(Me)₃ |
| 5 | Phenyl | H | H | Methyl | N(p-Tol)₂ |
| 6 | Phenyl | H | H | Methyl | Phenyl |
| 7 | Phenyl | H | H | tert-Butyl | H |
| 8 | Phenyl | H | H | tert-Buty | Methyl |
| 9 | Phenyl | H | H | tert-Butyl | tert-Butyl |
| 10 | Phenyl | H | H | tert-Butyl | Si(Me)₃ |
| 11 | Phenyl | H | H | tert-Butyl | N(p-Tol)₂ |
| 12 | Phenyl | H | H | tert-Butyl | Phenyl |
| 13 | Phenyl | H | H | Si(Me)₃ | H |
| 14 | Phenyl | H | H | Si(Me)₃ | Methyl |
| 15 | Phenyl | H | H | Si(Me)₃ | tert-Butyl |
| 16 | Phenyl | H | H | Si(Me)₃ | Si(Me)₃ |
| 17 | Phenyl | H | H | Si(Me)₃ | N(p-Tol)₂ |
| 18 | Phenyl | H | H | Si(Me)₃ | Phenyl |
| 19 | Phenyl | H | H | N(p-Tol)₂ | H |
| 20 | Phenyl | H | H | N(p-Tol)₂ | Methyl |
| 21 | Phenyl | H | H | N(p-Tol)₂ | tert-Butyl |
| 22 | Phenyl | H | H | N(p-Tol)₂ | Si(Me)₃ |
| 23 | Phenyl | H | H | N(p-Tol)₂ | N(p-Tol)₂ |
| 24 | Phenyl | H | H | N(p-Tol)₂ | Phenyl |
| 25 | Phenyl | H | H | Phenyl | H |
| 26 | Phenyl | H | H | Phenyl | Methyl |
| 27 | Phenyl | H | H | Phenyl | tert-Butyl |
| 28 | Phenyl | H | H | Phenyl | Si(Me)₃ |
| 29 | Phenyl | H | H | Phenyl | N(p-Tol)₂ |
| 30 | Phenyl | H | H | Phenyl | Phenyl |
| 31 | Phenyl | H | Methyl | Methyl | H |
| 32 | Phenyl | H | Methyl | Methyl | Methyl |
| 33 | Phenyl | H | Methyl | Methyl | tert-Butyl |
| 34 | Phenyl | H | Methyl | Methyl | Si(Me)₃ |
| 35 | Phenyl | H | Methyl | Methyl | N(p-Tol)₂ |
| 36 | Phenyl | H | Methyl | Methyl | Phenyl |
| 37 | Phenyl | H | Methyl | tert-Butyl | H |
| 38 | Phenyl | H | Methyl | tert-Butyl | Methyl |
| 39 | Phenyl | H | Methyl | tert-Butyl | tert-Butyl |
| 40 | Phenyl | H | Methyl | tert-Butyl | Si(Me)₃ |
| 41 | Phenyl | H | Methyl | tert-Butyl | N(p-Tol)₂ |
| 42 | Phenyl | H | Methyl | tert-Butyl | Phenyl |
| 43 | Phenyl | H | Methyl | Si(Me)₃ | H |
| 44 | Phenyl | H | Methyl | Si(Me)₃ | Methyl |
| 45 | Phenyl | H | Methyl | Si(Me)₃ | tert-Butyl |
| 46 | Phenyl | H | Methyl | Si(Me)₃ | Si(Me)₃ |
| 47 | Phenyl | H | Methyl | Si(Me)₃ | N(p-Tol)₂ |
| 48 | Phenyl | H | Methyl | Si(Me)₃ | Phenyl |
| 49 | Phenyl | H | Methyl | N(p-Tol)₂ | H |
| 50 | Phenyl | H | Methyl | N(p-Tol)₂ | Methyl |
| 51 | Phenyl | H | Methyl | N(p-Tol)₂ | tert-Butyl |
| 52 | Phenyl | H | Methyl | N(p-Tol)₂ | Si(Me)₃ |
| 53 | Phenyl | H | Methyl | N(p-Tol)₂ | N(p-Tol)₂ |
| 54 | Phenyl | H | Methyl | N(p-Tol)₂ | Phenyl |
| 55 | Phenyl | H | Methyl | Phenyl | H |
| 56 | Phenyl | H | Methyl | Phenyl | Methyl |
| 57 | Phenyl | H | Methyl | Phenyl | tert-Butyl |
| 58 | Phenyl | H | Methyl | Phenyl | Si(Me)₃ |
| 59 | Phenyl | H | Methyl | Phenyl | N(p-Tol)₂ |
| 60 | Phenyl | H | Methyl | Phenyl | Phenyl |
| 61 | Phenyl | H | tert-Butyl | Methyl | H |
| 62 | Phenyl | H | tert-Butyl | Methyl | Methyl |
| 63 | Phenyl | H | tert-Butyl | Methyl | tert-Butyl |
| 64 | Phenyl | H | tert-Butyl | Methyl | Si(Me)3 |
| 65 | Phenyl | H | tert-Butyl | Methyl | N(p-Tol)₂ |
| 66 | Phenyl | H | tert-Butyl | Methyl | Phenyl |
| 67 | Phenyl | H | tert-Butyl | tert-Butvl | H |
| 68 | Phenyl | H | tert-Butyl | tert-Butyl | Methyl |
| 69 | Phenyl | H | tert-Butyl | tert-Butyl | tert-Butyl |
| 70 | Phenyl | H | tert-Butyl | tert-Butyl | Si(Me)3. |
| 71 | Phenyl | H | tert-Butyl | tert-Butyl | N(p-Tol)₂ |
| 72 | Phenyl | H | tert-Butyl | tert-Butyl | Phenyl |
| 73 | Phenyl | H | tert-Butyl | Si(Me)₃ | H |
| 74 | Phenyl | H | tert-Butyl | Si(Me)₃ | Methyl |
| 75 | Phenyl | H | tert-Butyl | Si(Me)₃ | tert-Butyl |
| 76 | Phenyl | H | tert-Butyl | Si(Me)₃ | Si(Me)₃ |
| 77 | Phenyl | H | tert-Butyl | Si(Me)₃ | N(p-Tol)₂ |
| 78 | Phenyl | H | tert-Butyl | Si(Me)₃ | Phenyl |
| 79 | Phenyl | H | tert-Butyl | N(p-Tol)₂ | H |
| 80 | Phenyl | H | tert-Butyl | N(p-Tol)₂ | Methyl |
| 81 | Phenyl | H | tert-Butyl | N(p-Tol)₂ | tert-Butyl |
| 82 | Phenyl | H | tert-Butyl | N(p-Tol)₂ | Si(Me)₃ |
| 83 | Phenyl | H | tert-Butyl | N(p-Tol)₂ | N(p-Tol)₂ |
| 84 | Phenyl | H | tert-Buty | N(p-Tol)₂ | Phenyl |
| 85 | Phenyl | H | tert-Butvl | Phenyl | H |
| 86 | Phenyl | H | tert-Butyl | Phenyl | Methyl |
| 87 | Phenyl | H | tert-Butyl | Phenyl | tert-Butyl |
| 88 | Phenyl | H | tert-Butyl | Phenyl | Si(Me)₃ |
| 89 | Phenyl | H | tert-Butyl | Phenyl | N(p-Tol)₂ |
| 90 | Phenyl | H | tert-Butyl | Phenyl | Phenyl |
| 91 | Phenyl | H | Si(Me)₃ | Methyl | H |
| 92 | Phenyl | H | Si(Me)₃ | Methyl | Methyl |
| 93 | Phenyl | H | Si(Me)₃ | Methyl | tert-Butyl |
| 94 | Phenyl | H | Si(Me)₃ | Methyl | Si(Me)₃ |
| 95 | Phenyl | H | Si(Me)₃ | Methyl | N(p-Tol)₂ |
| 96 | Phenyl | H | Si(Me)₃ | Methyl | Phenyl |
| 97 | Phenyl | H | Si(Me)₃ | tert-Butyl | H |
| 98 | Phenyl | H | Si(Me)₃ | tert-Butyl | Methyl |
| 99 | Phenyl | H | Si(Me)₃ | tert-Butyl | tert-Butyl |
| 100 | Phenyl | H | Si(Me)₃ | tert-Butvl | Si(Me)₃ |
| 101 | Phenyl | H | Si(Me)₃ | tert-Butyl | N(p-Tol)₂ |
| 102 | Phenyl | H | Si(Me)₃ | tert-Butvl | Phenyl |
| 103 | Phenyl | H | Si(Me)₃ | Si(Me)₃ | H |
| 104 | Phenyl | H | Si(Me)₃ | Si(Me)₃ | Methyl |
| 105 | Phenyl | H | Si(Me)₃ | Si(Me)₃ | tert-Butyl |
| 106 | Phenyl | H | Si(Me)₃ | Si(Me)₃ | Si(Me)₃ |
| 107 | Phenyl | H | Si(Me)₃ | Si(Me)₃ | N(p-Tol)₂ |
| 108 | Phenyl | H | Si(Me)₃ | Si(Me)₃ | Phenyl |
| 109 | Phenyl | H | Si(Me)₃ | N(p-Tol)₂ | H |
| 110 | Phenyl | H | Si(Me)₃ | N(p-Tol)₂ | Methyl |
| 111 | Phenyl | H | Si(Me)₃ | N(p-Tol)₂ | tert-Butyl |
| 112 | Phenyl | H | Si(Me)₃ | N(p-Tol)₂ | Si(Me)₃ |
| 113 | Phenyl | H | Si(Me)₃ | N(p-Tol)₂ | N(p-Tol)₂ |
| 114 | Phenyl | H | Si(Me)₃ | N(p-Tol)₂ | Phenyl |
| 115 | Phenyl | H | Si(Me)₃ | Phenyl | H |
| 116 | Phenyl | H | Si(Me)₃ | Phenyl | Methyl |
| 117 | Phenyl | H | Si(Me)₃ | Phenyl | tert-Butyl |
| 118 | Phenyl | H | Si(Me)3 | Phenyl | Si(Me)₃ |
| 119 | Phenyl | H | Si(Me)₃ | Phenyl | N(p-Tol)₂ |
| 120 | Phenyl | H | Si(Me)₃ | Phenyl | Phenyl |
| 121 | Phenyl | H | N(p-Tol)₂ | Methyl | H |
| 122 | Phenyl | H | N(p-Tol)₂ | Methyl | Methyl |
| 123 | Phenyl | H | N(p-Tol)₂ | Methyl | tert-Butyl |
| 124 | Phenyl | H | N(p-Tol)₂ | Methyl | Si(Me)₃ |
| 125 | Phenyl | H | N(p-Tol)₂ | Methyl | N(p-Tol)₂ |
| 126 | Phenyl | H | N(p-Tol)₂ | Methyl | Phenyl |
| 127 | Phenyl | H | N(p-Tol)₂ | tert-Butyl | H |
| 128 | Phenyl | H | N(p-Tol)₂ | tert-Butyl | Methyl |
| 129 | Phenyl | H | N(p-Tol)₂ | tert-Butyl | tert-Butyl |
| 130 | Phenyl | H | N(p-Tol)₂ | tert-Butyl | Si(Me)₃ |
| 131 | Phenyl | H | N(p-Tol)₂ | tert-Butyl | N(p-Tol)₂ |
| 132 | Phenyl | H | N(p-Tol)₂ | tert-Butyl | Phenyl |
| 133 | Phenyl | H | N(p-Tol)₂ | Si(Me)₃ | H |
| 134 | Phenyl | H | N(p-Tol)₂ | Si(Me)₃ | Methyl |
| 135 | Phenyl | H | N(p-Tol)₂ | Si(Me)₃ | tert-Butyl |
| 136 | Phenyl | H | N(p-Tol)₂ | Si(Me)₃ | Si(Me)₃ |
| 137 | Phenyl | H | N(p-Tol)₂ | Si(Me)₃ | N(p-Tol)₂ |
| 138 | Phenyl | H | N(p-Tol)₂ | Si(Me)₃ | Phenyl |
| 139 | Phenyl | H | N(p-Tol)₂ | N(p-Tol)₂ | H |
| 140 | Phenyl | H | N(p-Tol)₂ | N(p-To)₂ | Methyl |
| 141 | Phenyl | H | N(p-Tol)₂ | N(p-Tol)₂ | tert-Butyl |
| 142 | Phenyl | H | N(p-Tol)₂ | N(p-Tol)₂ | Si(Me)₃ |
| 143 | Phenyl | H | N(p-Tol)₂ | N(p-Tol)₂ | N(p-Tol)₂ |
| 144 | Phenyl | H | N(p-Tol)₂ | N(p-Tol)₂ | Phenyl |
| 145 | Phenyl | H | N(p-Tol)₂ | Phenyl | H |
| 146 | Phenyl | H | N(p-Tol)₂ | Phenyl | Methyl |
| 147 | Phenyl | H | N(p-Tol)₂ | Phenyl | tert-Butvl |
| 148 | Phenyl | H | N(p-Tol)₂ | Phenyl | Si(Me)₃ |
| 149 | Phenyl | H | N(p-Tol)₂ | Phenyl | N(p-Tol)₂ |
| 150 | Phenyl | H | N(p-Tol)₂ | Phenyl | Phenyl |
| 151 | Phenyl | Methyl | H | Methyl | H |
| 152 | Phenyl | Methyl | H | Methyl | Methyl |
| 153 | Phenyl | Methyl | H | Methyl | tert-Butyl |
| 154 | Phenyl | Methyl | H | Methyl | Si(Me)₃ |
| 155 | Phenyl | Methyl | H | Methyl | N(p-Tol)₂ |
| 156 | Phenyl | Methyl | H | Methyl | Phenyl |
| 157 | Phenyl | Methyl | H | tert-Butyl | H |
| 158 | Phenyl | Methyl | H | tert-Butyl | Methyl |
| 159 | Phenyl | Methyl | H | tert-Butyl | tert-Butyl |
| 160 | Phenyl | Methyl | H | tert-Butyl | Si(Me)₃ |
| 161 | Phenyl | Methyl | H | tert-Butyl | N(p-Tol)₂ |
| 162 | Phenyl | Methyl | H | tert-Butyl | Phenyl |
| 163 | Phenyl | Methyl | H | Si(Me)₃ | H |
| 164 | Phenyl | Methyl | H | Si(Me)₃ | Methyl |
| 165 | Phenyl, | Methyl | H | Si(Me)₃ | tert-Butyl |
| 166 | Phenyl | Methyl | H | Si(Me)₃ | Si(Me)₃ |
| 167 | Phenyl | Methyl | H | Si(Me)₃ | N(p-Tol)₂ |
| 168 | Phenyl | Methyl | H | Si(Me)₃ | Phenyl |
| 169 | Phenyl | Methyl | H | N(p-Tol)₂ | H |
| 170 | Phenyl | Methyl | H | N(p-Tol)₂ | Methyl |
| 171 | Phenyl | Methyl | H | N(p-Tol)₂ | tert-Butyl |
| 172 | Phenyl | Methyl | H | N(p-Tol)₂ | Si(Me)₃ |
| 173 | Phenyl | Methyl | H | N(p-Tol)₂ | N(p-Tol)₂ |
| 174 | Phenyl | Methyl | H | N(p-Tol)₂ | Phenyl |
| 175 | Phenyl | Methyl | H | Phenyl | H |
| 176 | Phenyl | Methyl | H | Phenyl | Methyl |
| 177 | Phenyl | Methyl | H | Phenyl | tert-Butyl |
| 178 | Phenyl | Methyl | H | Phenyl | Si(Me)₃ |
| 179 | Phenyl | Methyl | H | Phenyl | N(p-Tol)₂ |
| 180 | Phenyl | Methyl | H | Phenyl | Phenyl |
| 181 | Phenyl | tert-Butyl | H | Methyl | H |
| 182 | Phenyl | tert-Butyl | H | Methyl | Methyl |
| 183 | Phenyl | tert-Butyl | H | Methyl | tert-Butyl |
| 184 | Phenyl | tert-Butyl | H | Methyl | Si(Me)₃ |
| 185 | Phenyl | tert-Butyl | H | Methyl | N(p-Tol)₂ |
| 186 | Phenyl | tert-Butyl | H | Methyl | Phenyl |
| 187 | Phenyl | tert-Butyl | H | tert-Butyl | H |
| 188 | Phenyl | tert-Butyl | H | tert-Butyl | Methyl |
| 189 | Phenyl | tert-Butyl | H | tert-Butyl | tert-Butyl |
| 190 | Phenyl | tert-Butyl | H | tert-Butyl | Si(Me)₃ |
| 191 | Phenyl | tert-Butyl | H | tert-Butyl | N(p-Tol)₂ |
| 192 | Phenyl | tert-Butyl | H | tert-Butvl | Phenyl |
| 193 | Phenyl | tert-Butyl | H | Si(Me)₃ | H |
| 194 | Phenyl | tert-Butyl | H | Si(Me)₃ | Methyl |
| 195 | Phenyl | tert-Butyl | H | Si(Me)₃ | tert-Butyl |
| 196 | Phenyl | tert-Butyl | H | Si(Me)₃ | Si(Me)₃ |
| 197 | Phenyl | tert-Butyl | H | Si(Me)3 | N(p-Tol)₂ |
| 198 | Phenyl | tert-Butyl | H | Si(Me)₃ | Phenyl |
| 199 | Phenyl | tert-Butyl | H | N(p-Tol)₂ | H |
| 200 | Phenyl | tert-Butyl | H | N(p-Tol)₂ | Methyl |
| 201 | Phenyl | tert-Butyl | H | N(p-Tol)₂ | tert-Butyl |
| 202 | Phenyl | tert-Butyl | H | N(p-Tol)₂ | Si(Me)₃ |
| 203 | Phenyl | tert-Butyl | H | N(p-Tol)₂ | N(p-Tol)₂ |
| 204 | Phenyl | tert-Butyl | H | N(p-Tol)₂ | Phenyl |
| 205 | Phenyl | tert-Butyl | H | Phenyl | H |
| 206 | Phenyl | tert-Butyl | H | Phenyl | Methyl |
| 207 | Phenyl | tert-Butyl | H | Phenyl | tert-Butyl |
| 208 | Phenyl | tert-Butyl | H | Phenyl | Si(Me)₃ |
| 209 | Phenyl | tert-Butyl | H | Phenyl | N(p-Tol)₂ |
| 210 | Phenyl | tert-Butyl | H | Phenyl | Phenyl |
| 211 | Phenyl | Si(Me)₃ | H | Methyl | H |
| 212 | Phenyl | Si(Me)₃ | H | Methyl | Methyl |
| 213 | Phenyl | Si(Me)₃ | H | Methyl | tert-Butyl |
| 214 | Phenyl | Si(Me)₃ | H | Methyl | Si(Me)₃ |
| 215 | Phenyl | Si(Me)₃ | H | Methyl | N(p-Tol)₂ |
| 216 | Phenyl | Si(Me)₃ | H | Methyl | Phenyl |
| 217 | Phenyl | Si(Me)₃ | H | tert-Butyl | H |
| 218 | Phenyl | Si(Me)₃ | H | tert-Butyl | Methyl |
| 219 | Phenyl | Si(Me)₃ | H | tert-Butyl | tert-Butyl |
| 220 | Phenyl | Si(Me)₃ | H | tert-Butyl | Si(Me)₃ |
| 221 | Phenyl | Si(Me)₃ | H | tert-Butyl | N(p-Tol)₂ |
| 222 | Phenvl | Si(Me)₃ | H | tert-Butyl | Phenyl |
| 223 | Phenyl | Si(Me)₃ | H | Si(Me)₃ | H |
| 224 | Phenyl | Si(Me)₃ | H | Si(Me)₃ | Methyl |
| 225 | Phenyl | Si(Me)₃ | H | Si(Me)₃ | tert-Butyl |
| 226 | Phenyl | Si(Me)₃ | H | Si(Me)₃ | Si(Me)₃ |
| 227 | Phenyl | Si(Me)₃ | H | Si(Me)₃ | N(p-Tol)₂ |
| 228 | Phenyl | Si(Me)3 | H | Si(Me)3 | Phenyl |
| 229 | Phenyl | Si(Me)₃ | H | Si(Me)₃ | H |
| 230 | Phenyl | Si(Me)₃ | H | N(p-Tol)₂ | Methyl |
| 231 | Phenyl | Si(Me)₃ | H | N(p-Tol)₂ | tert-Butyl |
| 232 | Phenyl | Si(Me)₃ | H | N(p-Tol)₂ | Si(Me)₃ |
| 233 | Phenyl | Si(Me)₃ | H | N(p-Tol)₂ | N(p-Tol)₂ |
| 234 | Phenyl | Si(Me)₃ | H | N(p-Tol)₂ | Phenyl |
| 235 | Phenyl | Si(Me)₃ | H | Phenyl | H |
| 236 | Phenyl | Si(Me)₃ | H | Phenyl | Methvl |
| 237 | Phenyl | Si(Me)₃ | H | Phenyl | tert-Butyl |
| 238 | Phenyl | Si(Me)₃ | H | Phenyl | Si(Me)₃ |
| 239 | Phenyl | Si(Me)₃ | H | Phenyl | N(p-Tol)₂ |
| 240 | Phenyl | Si(Me)₃ | H | Phenyl | Phenyl |
| 241 | Phenyl | N(p-Tol)₂ | H | Methyl | H |
| 242 | Phenyl | N(p-Tol)₂ | H | Methyl | Methyl |
| 243 | Phenyl | N(p-Tol)₂ | H | Methyl | tert-Buty |
| 244 | Phenyl | N(p-Tol)₂ | H | Methyl | Si(Me)₃ |
| 245 | Phenyl | N(p-Tol)₂ | H | Methyl | N(p-Tol)₂ |
| 246 | Phenyl | N(p-Tol)₂ | H | Methyl | Phenyl |
| 247 | Phenyl | N(p-Tol)₂ | H | tert-Butvl | H |
| 248 | Phenyl | N(p-Tol)₂ | H | tert-Butyl | Methyl |
| 249 | Phenyl | N(p-Tol)₂ | H | tert-Butyl | tert-Butyl |
| 250 | Phenyl | N(p-Tol)₂ | H | tert-Butyl | Si(Me)₃ |
| 251 | Phenyl | N(p-Tol)₂ | H | tert-Butyl | N(p-Tol)₂ |
| 252 | Phenyl | N(p-Tol)₂ | H | tert-Butyl | Phenyl |
| 253 | Phenyl | N(p-Tol)₂ | H | Si(Me)₃ | H |
| 254 | Phenyl | N(p-Tol)₂ | H | Si(Me)₃ | Methyl |
| 255 | Phenyl | N(p-Tol)₂ | H | Si(Me)₃ | tert-Butyl |
| 256 | Phenyl | N(p-Tol)₂ | H | Si(Me)₃ | Si(Me)₃ |
| 257 | Phenyl | N(p-Tol)₂ | H | Si(Me)₃ | N(p-Tol)₂ |
| 258 | Phenyl | N(p-Tol)₂ | H | Si(Me)₃ | Phenyl |
| 259 | Phenyl | N(p-Tol)₂ | H | N(p-Tol)₂ | H |
| 260 | Phenyl | N(p-Tol)₂ | H | N(p-Tol)₂ | Methyl |
| 261 | Phenyl | N(p-Tol)₂ | H | N(p-Tol)₂ | tert-Butyl |
| 262 | Phenyl | N(p-Tol)₂ | H | N(p-Tol)₂ | Si(Me)₃ |
| 263 | Phenyl | N(p-Tol)₂ | H | N(p-Tol)₂ | N(p-Tol)₂ |
| 264 | Phenyl | N(p-Tol)₂ | H | N(p-Tol)₂ | Phenyl |
| 265 | Phenyl | N(p-Tol)₂ | H | Phenyl | H |
| 266 | Phenyl | N(p-Tol)₂ | H | Phenyl | Methyl |
| 267 | Phenyl | N(p-Tol)₂ | H | Phenyl | tert-Butyl |
| 268 | Phenyl | N(p-Tol)₂ | H | Phenyl | Si(Me)₃ |
| 269 | Phenyl | N(p-Tol)₂ | H | Phenyl | N(p-Tol)₂ |
| 270 | Phenyl | N(p-Tol)₂ | H | Phenyl | Phenyl |
| 271 | Phenyl | Phenyl | H | Methyl | H |
| 272 | Phenyl | Phenyl | H | Methyl | Methyl |
| 273 | Phenyl | Phenyl | H | Methyl | tert-Butyl |
| 274 | Phenyl | Phenyl | H | Methyl | Si(Me)₃ |
| 275 | Phenyl | Phenyl | H | Methyl | N(p-Tol)₂ |
| 276 | Phenyl | Phenyl | H | Methyl | Phenyl |
| 277 | Phenyl | Phenyl | H | tert-Butyl | H |
| 278 | Phenyl | Phenyl | H | tert-Butyl | Methyl |
| 279 | Phenyl | Phenyl | H | tert-Butyl | tert-Butyl |
| 280 | Phenyl | Phenyl | H | tert-Butyl | Si(Me)₃ |
| 281 | Phenyl | Phenyl | H | tert-Butyl | N(p-Tol)₂ |
| 282 | Phenyl | Phenyl | H | tert-Butyl | Phenyl |
| 283 | Phenyl | Phenyl | H | Si(Me)₃ | H |
| 284 | Phenyl | Phenyl | H | Si(Me)₃ | Methyl |
| 285 | Phenyl | Phenyl | H | Si(Me)₃ | tert-Butyl |
| 286 | Phenyl | Phenyl | H | Si(Me)₃ | Si(Me)₃ |
| 287 | Phenyl | Phenyl | H | Si(Me)₃ | N(p-Tol)₂ |
| 288 | Phenyl | Phenyl | H | Si(Me)₃ | Phenyl |
| 289 | Phenyl | Phenyl | H | N(p-Tol)₂ | H |
| 290 | Phenyl | Phenyl | H | N(p-Tol)₂ | Methyl |
| 291 | Phenyl | Phenyl | H | N(p-Tol)₂ | tert-Butvl |
| 292 | Phenyl | Phenyl | H | N(p-Tol)₂ | Si(Me)₃ |
| 293 | Phenyl | Phenyl | H | N(p-Tol)₂ | N(p-Tol)₂ |
| 294 | Phenyl | Phenyl | H | N(p-Tol)₂ | Phenyl |
| 295 | Phenyl | Phenyl | H | Phenyl | H |
| 296 | Phenyl | Phenyl | H | Phenyl | Methyl |
| 297 | Phenyl | Phenyl | H | Phenyl | tert-Butyl |
| 298 | Phenyl | Phenyl | H | Phenyl | Si(Me)₃ |
| 299 | Phenyl | Phenyl | H | Phenyl | N(p-Tol)₂ |
| 300 | Phenyl | Phenyl | H | Phenyl | Phenyl |
| 301 | 1-Naphthyl | H | - | Methyl | H |
| 302 | 1-Naphthyl | H | - | Methyl | Methyl |
| 303 | 1-Naphthyl | H | - | Methyl | tert-Butyl |
| 304 | 1-Naphthyl | H | - | Methyl | Si(Me)₃ |
| 305 | 1-Naphthyl | H | - | Methyl | N(p-Tol)₂ |
| 306 | 1-Naphthyl | H | - | Methyl | Phenyl |
| 307 | 1-Naphthyl | H | - | tert-Butyl | H |
| 308 | 1-Naphthyl | H | - | tert-Butyl | Methyl |
| 309 | 1-Naphthyl | H | - | tert-Butyl | tert-Butyl |
| 310 | 1-Naphthyl | H | - | tert-Butyl | Si(Me)₃ |
| 311 | 1-Naphthyl | H | - | tert-Butyl | N(p-Tol)₂ |
| 312 | 1-Naphthyl | H | - | tert-Butyl | Phenyl |
| 313 | 1-Naphthyl | H | - | Si(Me)₃ | H |
| 314 | 1-Naphthyl | H | - | Si(Me)₃ | Methyl |
| 315 | 1-Naphthyl | H | - | Si(Me)₃ | tert-Butyl |
| 316 | 1-Naphthyl | H | - | Si(Me)₃ | Si(Me)₃ |
| 317 | 1-Naphthyl | H | - | Si(Me)₃ | N(p-Tol)₂ |
| 318 | 1-Naphthyl | H | - | Si(Me)₃ | N(p-Tol)₂ |
| 319 | 1-Naphthyl | H | - | N(p-Tol)₂ | H |
| 320 | 1-Naphthyl | H | - | N(p-Tol)₂ | Methyl |
| 321 | 1-Naphthyl | H | - | N(p-Tol)₂ | tert-Butyl |
| 322 | 1-Naphthyl | H | - | N(p-Tol)₂ | Si(Me)₃ |
| 323 | 1-Naphthyl | H | - | N(p-Tol)₂ | N(p-Tol)₂ |
| 324 | 1-Naphthyl | H | - | N(p-Tol)₂ | Phenyl |
| 325 | 1-Naphthyl | H | - | Phenyl | H |
| 326 | 1-Naphthyl | H | - | Phenyl | Methyl |
| 327 | 1-Naphthyl | H | - | Phenyl | tert-Butyl |
| 328 | 1-Naphthyl | H | - | Phenyl | Si(Me)₃ |
| 329 | 1-Naphthyl | H | - | Phenyl | N(p-Tol)₂ |
| 330 | 1-Naphthyl | H | - | Phenyl | Phenyl |
| 331 | 1-Naphthyl | Methyl | - | Methyl | H |
| 332 | 1-Naphthyl | Methyl | - | Methyl | Methyl |
| 333 | 1-Naphthvl | Methyl | - | Methyl | tert-Butyl |
| 334 | 1-Naphthyl | Methyl | - | Methyl | Si(Me)₃ |
| 335 | 1-Naphthyl | Methyl | - | Methyl | N(p-Tol)₂ |
| 336 | 1-Naphthyl | Methyl | - | Methyl | Phenyl |
| 337 | 1-Naphthyl | Methyl | - | tert-Butyl | H |
| 338 | 1-Naphthyl | Methyl | - | tert-Butyl | Methyl |
| 339 | 1-Naphthyl | Methyl | - | tert-Butyl | tert-Butyl |
| 340 | 1-Naphthyl | Methyl | - | tert-Butyl | Si(Me)₃ |
| 341 | 1-Naphthyl | Methyl | - | tert-Butyl | N(p-Tol)₂ |
| 342 | 1-Naphthyl | Methyl | - | tert-Butyl | Phenyl |
| 343 | 1-Naphthyl | Methyl | - | Si(Me)₃ | H |
| 344 | 1-Naphthyl | Methyl | - | Si(Me)₃ | Methyl |
| 345 | 1-Naphthyl | Methyl | - | Si(Me)₃ | tert-Butyl |
| 346 | 1-Naphthyl | Methyl | - | Si(Me)₃ | Si(Me)₃ |
| 347 | 1-Naphthyl | Methyl | - | Si(Me)₃ | N(p-Tol)₂ |
| 348 | 1-Naphthyl | Methyl | - | Si(Me)₃ | Phenyl |
| 349 | 1-Naphthyl | Methyl | - | N(p-Tol)₂ | H |
| 350 | 1-Naphthyl | Methyl | - | N(p-Tol)₂ | Methyl |
| 351 | 1-Naphthyl | Methyl | - | N(p-Tol)₂ | tert-Butyl |
| 352 | 1-Naphthyl | Methyl | - | N(p-Tol)₂ | Si(Me)₃ |
| 353 | 1-Naphthyl | Methyl | - | N(p-Tol)₂ | N(p-Tol)₂ |
| 354 | 1-Naphthyl | Methyl | - | N(p-Tol)₂ | Phenyl |
| 355 | 1-Naphthyl | Methyl | - | Phenyl | H |
| 356 | 1-Naphthyl | Methyl | - | Phenyl | Methyl |
| 357 | 1-Naphthyl | Methyl | - | Phenyl | tert-Butyl |
| 358 | 1-Naphthyl | Methyl | - | Phenyl | Si(Me)₃ |
| 359 | 1-Naphthyl | Methyl | - | Phenyl | N(p-Tol)₂ |
| 360 | 1-Naphthyl | Methyl | - | Phenyl | Phenyl |
| 361 | 2-Naphthyl | - | - | Methyl | H |
| 362 | 2-Naphthyl | - | - | Methyl | Methyl |
| 363 | 2-Naphthyl | - | - | Methyl | tert-Butyl |
| 364 | 2-Naphthyl | - | - | Methyl | Si(Me)₃ |
| 365 | 2-Naphthyl | - | - | Methyl | N(p-Tol)₂ |
| 366 | 2-Naphthyl | - | - | Methyl | Phenyl |
| 367 | 2-Naphthyl | - | - | tert-Butvl | H |
| 368 | 2-Naphthyl | - | - | tert-Butyl | Methyl |
| 369 | 2-Naphthyl | - | - | tert-Butyl | tert-Butyl |
| 370 | 2-Naphthyl | - | - | tert-Butyl | Si(Me)₃ |
| 371 | 2-Naphthyl | - | - | tert-Butyl | N(p-Tol)₂ |
| 372 | 2-Naphthyl | - | - | tert-Butyl | Phenyl |
| 373 | 2-Naphthyl | - | - | Si(Me)₃ | H |
| 374 | 2-Naphthyl | - | - | Si(Me)₃ | Methyl |
| 375 | 2-Naphthyl | - | - | Si(Me)₃ | tert-Butyl |
| 376 | 2-Naphthyl | - | - | Si(Me)₃ | Si(Me)₃ |
| 377 | 2-Naphthyl | - | - | Si(Me)₃ | N(p-Tol)₂ |
| 378 | 2-Naphthyl | - | - | Si(Me)₃ | Phenyl |
| 379 | 2-Naphthyl | - | - | N(p-Tol)₂ | H |
| 380 | 2-Naphthyl | - | - | N(p-Tol)₂ | Methyl |
| 381 | 2-Naphthyl | - | - | N(p-Tol)₂ | tert-Butyl |
| 382 | 2-Naphthyl | - | - | N(p-Tol)₂ | Si(Me)₃ |
| 383 | 2-Naphthyl | - | - | N(p-Tol)₂ | N(p-Tol)₂ |
| 384 | 2-Naphthyl | - | - | N(p-Tol)₂ | Phenyl |
| 385 | 2-Naphthyl | - | - | Phenyl | H |
| 386 | 2-Naphthyl | - | - | Phenyl | Methyl |
| 387 | 2-Naphthyl | - | - | Phenyl | tert-Butyl |
| 388 | 2-Naphthyl | - | - | Phenyl | Si(Me)₃ |
| 389 | 2-Naphthyl | - | - | Phenyl | N(p-Tol)₂ |
| 390 | 2-Naphthyl | - | - | Phenyl | Phenyl |
| 391 | 9-Anthryl | 1-Naphthyl | - | Methyl | H |
| 392 | 9-Anthryl | 1-Naphthyl | - | Methyl | Methyl |
| 393 | 9-Anthryl | 1-Naphthyl | - | Methyl | tert-Butyl |
| 394 | 9-Anthryl | 1-Naphthyl | - | Methyl | Si(Me)₃ |
| 395 | 9-Anthryl | 1-Naphthyl | - | Methyl | N(p-Tol)₂ |
| 396 | 9-Anthryl | 1-Naphthyl | - | Methyl | Phenyl |
| 397 | 9-Anthryl | 1-Naphthyl | - | tert-Butyl | H |
| 398 | 9-Anthryl | 1-Naphthyl | - | tert-Butyl | Methyl |
| 399 | 9-Anthryl | 1-Naphthyl | - | tert-Butyl | tert-Butyl |
| 400 | 9-Anthryl | 1-Naphthyl | - | tert-Butyl | Si(Me)₃ |
| 401 | 9-Anthryl | 1-Naphthyl | - | tert-Butyl | N(p-Tol)₂ |
| 402 | 9-Anthryl | 1-Naphthyl | - | tert-Butyl | Phenyl |
| 403 | 9-Anthryl | 1-Naphthyl | - | Si(Me)₃ | H |
| 404 | 9-Anthryl | 1-Naphthyl | - | Si(Me)₃ | Methyl |
| 405 | 9-Anthryl | 1-Naphthyl | - | Si(Me)₃ | tert-Butyl |
| 406 | 9-Anthryl | 1-Naphthyl | - | Si(Me)₃ | Si(Me)₃ |
| 407 | 9-Anthryl | 1-Naphthyl | - | Si(Me)₃ | N(p-Tol)₂ |
| 408 | 9-Anthryl | 1-Naphthyl | - | Si(Me)₃ | Phenyl |
| 409 | 9-Anthryl | 1-Naphthyl | - | N(p-Tol)₂ | H |
| 410 | 9-Anthryl | 1-Naphthyl | - | N(p-Tol)₂ | Methyl |
| 411 | 9-Anthryl | 1-Naphthyl | - | N(p-Tol)₂ | term-butyl |
| 412 | 9-Anthryl | 1-Naphthyl | - | N(p-Tol)₂ | Si(Me)₃ |
| 413 | 9-Anthryl | 1-Naphthyl | - | N(p-Tol)₂ | N(p-Tol)₂ |
| 414 | 9-Anthryl | 1-Naphthyl | - | N(p-Tol)₂ | Phenyl |
| 415 | 9-Anthryl | 1-Naphthyl | - | Phenyl | H |
| 416 | 9-Anthryl | 1-Naphthyl | - | Phenyl | Methyl |
| 417 | 9-Anthrvl | 1-Naphthyl | - | Phenyl | tert-Butyl |
| 418 | 9-Anthryl | 1-Naphthyl | - | Phenyl | Si(Me)₃ |
| 419 | 9-Anthryl | 1-Naphthyl | - | Phenyl | N(p-Tol)₂ |
| 420 | 9-Anthryl | 1-Naphthyl | - | Phenyl | Phenyl |
| 421 | 9-Anthryl | 2-Naphthyl | - | Methyl | H |
| 422 | 9-Anthryl | 2-Naphthyl | - | Methyl | Methyl |
| 423 | 9-Anthryl | 2-Naphthyl | - | Methyl | tert-Butyl |
| 424 | 9-Anthryl | 2-Naphthyl | - | Methyl | Si(Me)₃ |
| 425 | 9-Anthryl | 2-Naphthyl | - | Methyl | N(p-Tol)₂ |
| 426 | 9-Anthryl | 2-Naphthyl | - | Methyl | Phenyl |
| 427 | 9-Anthryl | 2-Naphthyl | - | tert-Butyl | H |
| 428 | 9-Anthryl | 2-Naphthyl | - | tert-Butyl | Methyl |
| 429 | 9-Anthryl | 2-Naphthyl | - | tert-Butyl | tert-Butyl |
| 430 | 9-Anthryl | 2-Naphthyl | - | tert-Butyl | Si(Me)₃ |
| 431 | 9-Anthryl | 2-Naphthyl | - | tert-Butyl | N(p-Tol)₂ |
| 432 | 9-Anthryl | 2-Naphthyl | - | tert-Butyl | Phenyl |
| 433 | 9-Anthryl | 2-Naphthyl | - | Si(Me)₃ | H |
| 434 | 9-Anthryl | 2-Naphthyl | - | Si(Me)₃ | Methyl |
| 435 | 9-Anthryl | 2-Naphthyl | - | Si(Me)₃ | tert-Butyl |
| 436 | 9-Anthryl | 2-Naphthyl | - | Si(Me)₃ | Si(Me)₃ |
| 437 | 9-Anthryl | 2-Naphthyl | - | Si(Me)₃ | N(p-Tol)₂ |
| 438 | 9-Anthryl | 2-Naphthyl | - | Si(Me)₃ | Phenyl |
| 439 | 9-Anthryl | 2-Naphthyl | - | N(p-Tol)₂ | H |
| 440 | 9-Anthryl | 2-Naphthyl | - | N(p-Tol)₂ | Methyl |
| 441 | 9-Anthryl | 2-Naphthyl | - | N(p-Tol)₂ | tert-Butyl |
| 442 | 9-Anthryl | 2-Naphthyl | - | N(p-Tol)₂ | Si(Me)₃ |
| 443 | 9-Anthryl | 2-Naphthyl | - | N(p-Tol)₂ | N(p-Tol)₂ |
| 444 | 9-Anthryl | 2-Naphthyl | - | N(p-Tol)₂ | Phenyl |
| 445 | 9-Anthryl | 2-Naphthyl | - | Phenyl | H |
| 446 | 9-Anthryl | 2-Naphthyl | - | Phenyl | Methyl |
| 447 | 9-Anthryl | 2-Naphthyl | - | Phenyl | tert-Butyl |
| 448 | 9-Anthryl | 2-Naphthyl | - | Phenyl | Si(Me)₃ |
| 449 | 9-Anthryl | 2-Naphthyl | - | Phenyl | N(p-Tol)₂ |
| 450 | 9-Anthryl | 2-Naphthyl | - | Phenyl | Phenyl |
| 451 | 9-Anthryl | N(p-Tol)₂ | - | Methyl | H |
| 452 | 9-Anthryl | N(p-Tol)₂ | - | Methyl | Methyl |
| 453 | 9-Anthryl | N(p-Tol)₂ | - | Methyl | tert-Butyl |
| 454 | 9-Anthryl | N(p-Tol)₂ | - | Methyl | Si(Me)₃ |
| 455 | 9-Anthryl | N(p-Tol)₂ | - | Methyl | N(p-Tol)₂ |
| 456 | 9-Anthryl | N(p-Tol)₂ | - | Methyl | Phenyl |
| 457 | 9-Anthryl | N(p-Tol)₂ | - | tert-Butyl | H |
| 458 | 9-Anthryl | N(p-Tol)₂ | - | tert-Butyl | Methyl |
| 459 | 9-Anthryl | N(p-Tol)₂ | - | tert-Butyl | tert-Butyl |
| 460 | 9-Anthryl | N(p-Tol)₂ | - | tert-Butyl | Si(Me)₃ |
| 461 | 9-Anthryl | N(p-Tol)₂ | - | tert-Butyl | N(p-Tol)₂ |
| 462 | 9-Anthryl | N(p-Tol)₂ | - | tert-Butyl | Phenyl |
| 463 | 9-Anthryl | N(p-Tol)₂ | - | Si(Me)₃ | H |
| 464 | 9-Anthryl | N(p-Tol)₂ | - | Si(Me)₃ | Methyl |
| 465 | 9-Anthryl | N(p-Tol)₂ | - | Si(Me)₃ | tert-Butyl |
| 466 | 9-Anthryl | N(p-Tol)₂ | - | Si(Me)₃ | Si(Me)₃ |
| 467 | 9-Anthryl | N(p-Tol)₂ | - | Si(Me)₃ | N(p-Tol)₂ |
| 468 | 9-Anthryl | N(p-Tol)₂ | - | Si(Me)₃ | Phenyl |
| 469 | 9-Anthryl | N(p-Tol)₂ | - | N(p-Tol)₂ | H |
| 470 | 9-Anthryl | N(p-Tol)₂ | - | N(p-Tol)₂ | Methyl |
| 471 | 9-Anthryl | N(p-Tol)₂ | - | N(p-Tol)₂ | tert-Butyl |
| 472 | 9-Anthryl | N(p-Tol)₂ | - | N(p-Tol)₂ | Si(Me)₃ |
| 473 | 9-Anthryl | N(p-Tol)₂ | - | N(p-Tol)₂ | N(p-Tol)₂ |
| 474 | 9-Anthryl | N(p-Tol)₂ | - | N(p-Tol)₂ | Phenyl |
| 475 | 9-Anthryl | N(p-Tol)₂ | - | Phenyl | H |
| 476 | 9-Anthryl | N(p-Tol)₂ | - | Phenyl | Methyl |
| 477 | 9-Anthryl | N(p-Tol)₂ | - | Phenyl | tert-Butvl |
| 478 | 9-Anthryl | N(p-Tol)₂ | - | Phenyl | Si(Me)₃ |
| 479 | 9-Anthryl | N(p-Tol)₂ | - | Phenyl | N(p-Tol)₂ |
| 480 | 9-Anthryl | N(p-Tol)₂ | - | Phenyl | Phenyl |
| 481 | 9-Anthryl | Phenyl | - | Methyl | H |
| 482 | 9-Anthryl | Phenyl | - | Methyl | Methyl |
| 483 | 9-Anthryl | Phenyl | - | Methyl | tert-Butyl |
| 484 | 9-Anthryl | Phenyl | - | Methyl | Si(Me)₃ |
| 485 | 9-Anthryl | Phenyl | - | Methyl | N(p-Tol)₂ |
| 486 | 9-Anthryl | Phenyl | - | Methyl | Phenyl |
| 487 | 9-Anthryl | Phenyl | - | tert-Butyl | H |
| 488 | 9-Anthrvl | Phenyl | - | tert-Butyl | Methyl |
| 489 | 9-Anthryl | Phenyl | - | tert-Butyl | tert-Butyl |
| 490 | 9-Anthryl | Phenyl | - | tert-Butyl | Si(Me)₃ |
| 491 | 9-Anthryl | Phenyl | - | tert-Butyl | N(p-Tol)₂ |
| 492 | 9-Anthryl | Phenyl | - | tert-Butyl | Phenyl |
| 493 | 9-Anthryl | Phenyl | - | Si(Me)₃ | H |
| 494 | 9-Anthryl | Phenyl | - | Si(Me)₃ | Methyl |
| 495 | 9-Anthryl | Phenyl | - | Si(Me)₃ | tert-Butyl |
| 496 | 9-Anthryl | Phenyl | - | Si(Me)₃ | Si(Me)₃ |
| 497 | 9-Anthryl | Phenyl | - | Si(Me)₃ | N(p-Tol)₂ |
| 498 | 9-Anthryl | Phenyl | - | Si(Me)₃ | Phenyl |
| 499 | 9-Anthryl | Phenyl | - | N(p-Tol)₂ | H |
| 500 | 9-Anthryl | Phenyl | - | N(p-Tol)₂ | Methyl |
| 501 | 9-Anthryl | Phenyl | - | N(p-Tol)₂ | tert-Butyl |
| 502 | 9-Anthryl | Phenyl | - | N(p-Tol)₂ | Si(Me)₃ |
| 503 | 9-Anthryl | Phenyl | - | N(p-Tol)₂ | N(p-Tol)₂ |
| 504 | 9-Anthryl | Phenyl | - | N(p-Tol)₂ | Phenyl |
| 505 | 9-Anthryl | Phenyl | - | Phenyl | H |
| 506 | 9-Anthryl | Phenyl | - | Phenyl | Methyl |
| 507 | 9-Anthryl | Phenyl | - | Phenyl | tert-Butyl |
| 508 | 9-Anthryl | Phenyl | - | Phenyl | Si(Me)₃ |
| 509 | 9-Anthryl | Phenyl | - | Phenyl | N(p-Tol)₂ |
| 510 | 9-Anthryl | Phenyl | - | Phenyl | Phenyl |

Die Synthese der Verbindungen ist beispielsweise möglich ausgehend von 2,6-Dichlor- oder Dibromanthrachinon. Dieses wird in einer Suzuki-Kupplung mit Arylboronsäuren zum entsprechenden 2,6-Diarylanthrachinon umgesetzt. Dieses kann in einem weiteren Schritt mit einem aromatischen Grignard-Reagenz und dann mit einem Reduktionsmittel, beispielsweise Zinn(II)chlorid, zum 2,6,9,10-Tetraarylanthracen umgesetzt werden.

Ein weiterer Gegenstand der vorliegenden Erfindung ist daher ein Verfahren zur Herstellung von Verbindungen gemäß Formel (1) durch Reaktion von 2,6-Dihalogenanthrachinon oder einem analogen Sulfonsäurederivat mit einem Boronsäurederivat der Gruppe Ar unter Palladium-Katalyse, gefolgt von Umsetzung mit einem entsprechenden ortho-substituierten metallorganischen Phenylderivat und Reduktion.

Das Verfahren erfolgt also nach dem folgenden Schema:

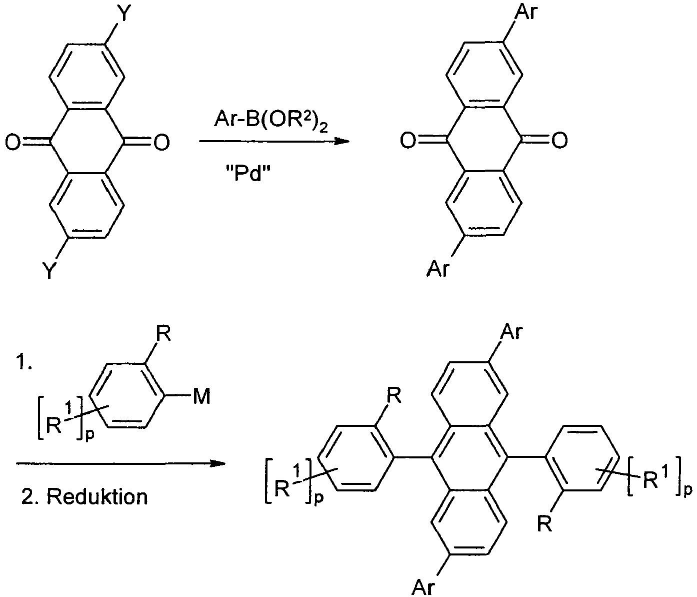

Dabei haben Ar, R, R¹, R² und p dieselben Bedeutungen, wie oben beschrieben. Y steht für ein Chlor, Brom oder Iod, bevorzugt Brom, oder für eine Gruppe der Formel OSO₂R². M steht für ein elektropositives Metall, insbesondere Lithium, Magnesium oder Zink, und enthält im Fall eines zweiwertigen Metalls noch eine weitere organische Gruppe oder eine Gruppe Y. Wie eine Suzuki-Kupplung (erster Reaktionsschritt) durchgeführt wird und welche Palladiumkatalysatoren sich hierfür besonders eignen, ist dem Fachmann der organischen Synthese bekannt. Als Reduktionsmittel im zweiten Reaktionsschritt wird bevorzugt Zinn(II)chlorid eingesetzt.

Die Verbindungen gemäß Formel (1) können in organischen Elektrolumineszenzvorrichtungen eingesetzt werden. Dabei eignen sie sich insbesondere für die Verwendung als Hostmaterial für fluoreszierende Emitter, können aber je nach Substitutionsmuster auch als Emitter, als Lochtransportmaterial, als Lochblockiermaterial und/oder als Elektronentransportmaterial eingesetzt werden.

Weiterer Gegenstand der Erfindung ist daher die Verwendung von Verbindungen gemäß Formel (1) in organischen elektronischen Vorrichtungen, insbesondere in organischen Elektrolumineszenzvorrichtungen, insbesondere als Hostmaterial, als Emitter, als Lochtransportmaterial, als Lochblockiermaterial und/oder als Elektronentransportmaterial.

Weiterer Gegenstand der Erfindung sind organische elektronische Vorrichtungen, insbesondere organische Elektrolumineszenzvorrichtungen, enthaltend Anode, Kathode und mindestens eine emittierende Schicht, wobei mindestens eine Schicht mindestens eine Verbindung gemäß Formel (1) enthält. Bevorzugt ist die Schicht, die die Verbindung gemäß Formel (1) enthält, eine emittierende Schicht, eine Lochtransportschicht, eine Lochinjektionsschicht, eine Lochblockierschicht oder eine Elektronentransportschicht.

Außer Kathode, Anode und der emittierenden Schicht (bzw. den emittierenden Schichten) kann die organische Elektrolumineszenzvorrichtung noch weitere Schichten enthalten. Diese können beispielsweise sein: Lochinjektionsschicht, Lochtransportschicht, Lochblockierschicht, Elektronentransportschicht, Elektroneninjektionsschicht und/oder eine Charge-Generation Layer (T. Matsumoto et al., Multiphoton Organic EL Device Having Charge Generation Layer, IDMC 2003, Taiwan; Session 21 OLED (5)). Die Materialien in diesen Schichten können auch dotiert sein. Es muss nicht notwendigerweise jede dieser Schichten vorhanden sein. Als Lochtransportmaterialien eignen sich beispielsweise aromatische Amine, wie sie üblicherweise gemäß dem Stand der Technik verwendet werden, welche auch p-dotiert sein können. Als Elektronentransportmaterialien eignen sich beispielsweise Metallchelatkomplexe, z. B. AlQ₃, Verbindungen auf Basis elektronenarmer Heterocyclen, z. B. Triazinderivate, oder Verbindungen enthaltend aromatische Carbonyle oder Phosphinoxide, wie z. B. beschrieben in WO 05/084081 und WO 05/084082, welche jeweils auch n-dotiert sein können. Als Elektroneninjektionsmaterialien eignen sich insbesondere Fluoride und Oxide der Alkali- und Erdalkalimetalle, beispielsweise NaF, BaF₂, CaF₂, LiF oder Li₂O.

In einer bevorzugten Ausführungsform der Erfindung wird die Verbindung gemäß Formel (1) als Hostmaterial, insbesondere für fluoreszierende Emitter, und/oder als Elektronentransportmaterial und/oder als Lochblockiermaterial eingesetzt. Dies ist insbesondere dann der Fall, wenn die Verbindung keinen Substituenten der Formel N(Ar¹)₂ enthält.

Unter einem Hostmaterial wird in einem System aus Host und Dotand (binäre Mischung) diejenige Komponente verstanden, die in dem System im höheren Anteil vorliegt. Bei einem System aus einem Host und mehreren Dotanden (ternäre und höhere Mischungen) wird als Host diejenige Komponente verstanden, deren Anteil der höchste in der Mischung ist.

Der Anteil des Hostmaterials gemäß der Formel (1) in der emittierenden Schicht beträgt zwischen 50.0 und 99.9 Gew.-%, bevorzugt zwischen 80.0 und 99.5 Gew.-%, besonders bevorzugt zwischen 90.0 und 99.0 Gew.-%. Entsprechend beträgt der Anteil des Dotanden in der emittierenden Schicht zwischen 0.1 und 50.0 Gew.-%, bevorzugt zwischen 0.5 und 20.0 Gew.-%, besonders bevorzugt zwischen 1.0 und 10.0 Gew.-%.

Bevorzugte Dotanden sind ausgewählt aus der Klasse der aromatischen Anthracenamine, der aromatischen Anthracendiamine, der aromatischen Pyrenamine, der aromatischen Pyrendiamine, der Monostyrylamine, der Distyrylamine, der Tristyrylamine, der Tetrastyrylamine, der Styrylphosphine, der Styrylether und der Arylamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine und Pyrendiamine sind analog dazu definiert, wobei die Diarylaminogruppen am Pyren bevorzugt in 1-Position bzw. in 1,6-Position gebunden sind. Unter einem Monostyrylamin wird eine Verbindung verstanden, die eine substituierte oder unsubstituierte Styrylgruppe und mindestens ein, bevorzugt aromatisches, Amin enthält. Unter einem Distyrylamin wird eine Verbindung verstanden, die zwei substituierte oder unsubstituierte Styrylgruppen und mindestens ein, bevorzugt aromatisches, Amin enthält. Unter einem Tristyrylamin wird eine Verbindung verstanden, die drei substituierte oder unsubstituierte Styrylgruppen und mindestens ein, bevorzugt aromatisches, Amin enthält. Unter einem Tetrastyrylamin wird eine Verbindung verstanden, die vier substituierte oder unsubstituierte Styrylgruppen und mindestens ein, bevorzugt aromatisches, Amin enthält. Entsprechende Phosphine und Ether sind in Analogie zu den Aminen definiert. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist dabei mindestens eine Arylgruppe eine kondensierte Arylgruppe mit mindestens drei Ringen. Die Styrylgruppen sind besonders bevorzugt Stilbene, die auch noch weiter substituiert sein können. Besonders bevorzugte Dotanden sind gewählt aus den Klassen der Tristilbenamine, der aromatischen Stilbendiamine, der Anthracendiamine und der Pyrendiamine. Ganz besonders bevorzugte Dotanden sind ausgewählt aus der Klasse der Tristyrylamine. Beispiele für derartige Dotanden sind substituierte oder unsubstituierte Tristilbenamine oder die Dotanden, die in WO 06/000388, WO 06/058737 und WO 06/000389 beschrieben sind.

In einer weiteren Ausführungsform der Erfindung enthält die organische Elektrolumineszenzvorrichtung mehrere emittierende Schichten, wobei mindestens eine dieser Schichten mindestens eine Verbindung gemäß Formel (1) enthält. Besonders bevorzugt weisen diese Emissionsschichten insgesamt mehrere Emissionsmaxima zwischen 380 nm und 750 nm auf, so dass insgesamt weiße Emission resultiert, d. h. in der bzw. den weiteren emittierenden Schichten wird noch mindestens eine weitere emittierende Verbindung verwendet, die fluoreszieren oder phosphoreszieren kann und die gelbes, orange oder rotes Licht emittiert. Bevorzugt sind Dreischichtsysteme, wobei mindestens eine dieser Schichten mindestens eine Verbindung gemäß Formel (1) enthält und wobei die drei Schichten blaue, grüne und orange oder rote Emission zeigen (für den prinzipiellen Aufbau siehe z. B. WO 05/011013). Ebenso eignen sich für weiße Emission Emitter, welche breitbandige Emissionsbanden aufweisen und dadurch weiße Emission zeigen.

Außer den Verbindungen gemäß Formel (1) und dem bzw. den Dotanden können in der emittierenden Schicht auch weitere Substanzen vorhanden sein, beispielsweise Loch- oder Elektronentransportmaterialien.

Wenn das Symbol R für eine Gruppe N(Ar¹)₂ steht und/oder mindestens ein Substituent R¹ an der Gruppe Ar oder an einer anderen Position für eine Gruppe N(Ar¹)₂ steht, eignet sich die Verbindung gemäß Formel (1) besonders gut als emittierende Verbindung und/oder als Lochtransportmaterial, wie im Folgenden genauer ausgeführt.

Wenn die Verbindung der Formel (1) als Lochtransportmaterial eingesetzt wird, wird sie bevorzugt in einer Lochtransportschicht und/oder in einer Lochinjektionsschicht eingesetzt. Eine Lochinjektionsschicht im Sinne dieser Erfindung ist eine Schicht, die direkt an die Anode angrenzt. Eine Lochtransportschicht im Sinne dieser Erfindung ist eine Schicht, die zwischen einer Lochinjektionsschicht und einer Emissionsschicht liegt. Wenn die Verbindungen gemäß Formel (1) als Lochtransport- bzw. als Lochinjektionsmaterial verwendet werden, kann es bevorzugt sein, wenn sie mit Elektronenakzeptor-Verbindungen dotiert ist, beispielsweise mit F₄-TCNQ oder mit Verbindungen, wie sie in EP 1476881 oder EP 1596445 beschrieben werden.

Wenn die Verbindung der Formel (1) als emittierende Verbindung eingesetzt wird, wird sie bevorzugt in Kombination mit einem Hostmaterial eingesetzt.

Der Anteil der emittierenden Verbindung gemäß Formel (1) in der Mischung der emittierenden Schicht beträgt zwischen 0.1 und 50.0 Gew.-%, bevorzugt zwischen 0.5 und 20.0 Gew. %, besonders bevorzugt zwischen 1.0 und 10.0 Gew.-%. Entsprechend beträgt der Anteil des Hostmaterials in der Schicht zwischen 50.0 und 99.9 Gew.-%, bevorzugt zwischen 80.0 und 99.5 Gew.-%, besonders bevorzugt zwischen 90.0 und 99.0 Gew.-%.

Als Hostmaterialien kommen verschiedene Stoffklassen in Frage. Bevorzugte Hostmaterialien sind ausgewählt aus den Klassen der Oligoarylene (z. B. 2,2',7,7'-tetraphenyl-spirobifluoren gemäß EP 676461 oder Dinaphthylanthracen), insbesondere der Oligo-arylene enthaltend kondensierte aromatische Gruppen, der Oligo-arylenvinylene (z. B. DPVBi oder Spiro-DPVBi gemäß EP 676461), der polypodalen Metallkomplexe (z. B. gemäß WO 04/081017), der lochleitenden Verbindungen (z. B. gemäß WO 04/058911), der elektronenleitenden Verbindungen, insbesondere Ketone, Phosphinoxide, Sulfoxide, etc. (z. B. gemäß WO 05/084081 oder WO 05/084082), der Atropisomere (z. B. gemäß WO 06/048268) oder der Boronsäurederivate (z. B. gemäß WO 06/117052). Besonders bevorzugte Hostmaterialien sind ausgewählt aus den Klassen der Oligoarylene, enthaltend Naphthalin, Anthracen und/oder Pyren oder Atropisomeren dieser Verbindungen, der Oligo-arylenvinylene, der Ketone, der Phosphinoxide und der Sulfoxide. Ganz besonders bevorzugte Hostmaterialien sind ausgewählt aus den Klassen der Oligoarylene, enthaltend Anthracen und/oder Pyren oder Atropisomeren dieser Verbindungen, der Phosphinoxide und der Sulfoxide.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Druck kleiner 10⁻⁵ mbar, bevorzugt kleiner 10⁻⁶ mbar, besonders bevorzugt kleiner 10⁻⁷ mbar aufgedampft.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (1) nötig. Daher eignen sich die erfindungsgemäßen Verbindungen sehr gut für die Verarbeitung aus Lösung, da sie durch die Substitution hohe Löslichkeit in organischen Lösemitteln aufweisen.

Die erfindungsgemäßen organischen Elektrolumineszenzvorrichtungen weisen folgende überraschende Eigenschaften auf:
1. Die erfindungsgemäßen Verbindungen weisen eine hohe thermische Stabilität und insbesondere eine hohe Glasübergangstemperatur auf.
2. Die erfindungsgemäßen Verbindungen zeigen bei Verwendung in OLEDs hohe Effizienz, gute Lebensdauer und gute Farbkoordinaten.
3. Die erfindungsgemäßen Verbindungen weisen eine gute Löslichkeit in organischen Lösemitteln auf, was die Herstellung und Verarbeitung dieser Verbindungen vereinfacht.
4. Die erfindungsgemäßen Verbindungen weisen eine hohe Redoxstabilität (hohe Stabilität gegenüber Löchern und Elektronen) auf.
5. Die Filmbildungseigenschaften der erfindungsgemäßen Verbindungen sind sehr gut.

Im vorliegenden Anmeldetext wird auf die Verwendung erfindungsgemäßer Verbindungen in Bezug auf OLEDs und die entsprechenden Displays abgezielt.

Trotz dieser Beschränkung der Beschreibung ist es für den Fachmann ohne weiteres erfinderisches Zutun möglich, die erfindungsgemäßen Verbindungen auch für weitere Verwendungen in anderen elektronischen Vorrichtungen zu benutzen, z. B. für organische Feld-Effekt-Transistoren (O-FETs), organische Dünnfilmtransistoren (O-TFTs), organische lichtemittierende Transistoren (O-LETs), organische integrierte Schaltungen (O-ICs), organische Solarzellen (O-SCs), organische Feld-Quench-Devices (O-FQDs), organische Photorezeptoren, lichtemittierende elektrochemische Zellen (LECs) oder auch organische Laserdioden (O-Laser), um nur einige Anwendungen zu nennen.

Die Verwendung der erfindungsgemäßen Verbindungen in den entsprechenden Vorrichtungen ebenso wie diese Vorrichtungen selbst sind ein weiterer Gegenstand der vorliegenden Erfindung.

Die Erfindung wird durch die nachfolgenden Beispiele näher erläutert, ohne sie dadurch einschränken zu wollen.

### Beispiele:

Die nachfolgenden Synthesen werden, sofern nicht anders angegeben, unter einer Schutzgasatmosphäre durchgeführt. Die Edukte können von den Firmen ALDRICH bzw. ABCR (Palladium(II)acetat, Tri-o-tolylphosphin, Di-tert-butyl-chlorphosphin, Bromide, Amine, Anorganika, Lösemittel) bezogen werden. 2,6-Dibrom-anthrachinon wird nach Lee et al., Org. Lett. 2005, 7(2), 323 dargestellt; 2-Trimethylsilylbrombenzol wird nach Klusener et al., Org. Chem. 1990, 55(4), 1311 dargestellt; 10-(4-Methylnaphth-1-yl)anthracen-9-boronsäurepinacolester wird nach EP 05009643.7 dargestellt; 1-Brom-2-(1-methyl-1-phenylethyl)benzol wird nach Sigmundova et al., Synth. Commun. 2004, 34(20), 3667 dargestellt.

### Beispiel 1: 2,6,9,10-Tetra-o-tolyl-anthracen

### a) 2,6-Bis-o-tolyl-anthrachinon

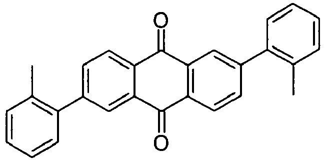

Eine Suspension von 28.7 g (100 mmol) 2,6-Dibrom-anthrachinon, 32.6 g (240 mmol) o-Tolylboronsäure, 89.2 g (420 mmol) Kaliumphosphat, 1.8 g (6 mmol) Tri-o-tolylphosphin und 225 mg (1 mmol) Palladium(II)acetat in einem Gemisch aus 200 ml Dioxan, 400 ml Toluol und 500 ml Wasser wird 16 h unter Rückfluss erhitzt. Nach Erkalten wird der Feststoff abgesaugt, dreimal mit je 100 ml Wasser, dreimal mit je 100 ml Ethanol gewaschen, im Vakuum getrocknet und anschließend zweimal aus DMF umkristallisiert. Ausbeute: 33.0 g (85 mmol), 84.9 % d. Th., Reinheit: 98 % n. NMR.

### b) 2,6,9,10-Tetra-o-tolyl-anthracen

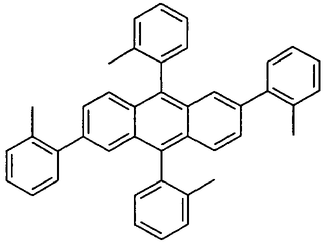

Aus 3.7 g (153 mmol) Magnesium und 18.0 ml (150 mmol) 2-Bromtoluol in 500 ml THF wird das entsprechende Grignardreagenz dargestellt. Das Grignardreagenz wird mit 19.4 g (50 mmol) 2,6-Bis-o-tolyl-anthrachinon versetzt. Anschließend wird die Reaktionsmischung 16 h unter Rückfluss erhitzt. Nach Erkalten wird mit 30 ml Ethanol versetzt, das Lösemittel wird im Vakuum entfernt, der Rückstand wird in 300 ml DMF aufgenommen, auf 60 °C erwärmt und dann unter gutem Rühren portionsweise (Achtung: exotherme Reaktion!) mit 8.9 g (65 mmol) Zinn(II)chlorid versetzt. Anschließend wird noch 2 h bei 60 °C nachgerührt. Nach Erkalten wird mit 500 ml 2.5 N Salzsäure versetzt und vom Feststoff abgesaugt. Der Feststoff wird dreimal mit je 100 ml 2.5 N Salzsäure, dreimal mit je 100 ml Wasser und dreimal mit je 100 ml Ethanol gewaschen, im Vakuum getrocknet, anschließend dreimal aus Essigsäure und zweimal aus DMF umkristallisiert. Sublimation p = 1 x 10⁻⁵ mbar, T = 335 °C. Ausbeute: 19.8 g (37 mmol), 73.5 % d. Th.; Reinheit: 99.8 % n. HPLC. Mischung aus zwei Atropisomeren n. ¹H-NMR-Spektroskopie.

### Beispiel 2: 2,6-Bis-o-tolyl-9,10-bis-(2-(1-methyl-1-phenylethyl)phenyl)-anthracen

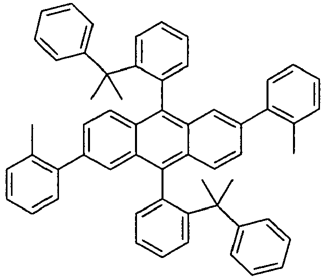

Durchführung analog Beispiel 1 b. Anstelle von 18.0 ml (150 mmol) 2-Bromtoluol werden 41.3 g (150 mmol) 1-Brom-2-(1-methyl-1-phenyl-ethyl)-benzol verwendet. Umkristallisation aus Dioxan. Sublimation p = 1 x 10⁻⁵ mbar, T = 360 °C. Ausbeute: 22.6 g (30 mmol), 60.5 % d. Th.; Reinheit: 99.9 % n. HPLC. Atropisomerenrein n. ¹H-NMR-Spektroskopie.

### Beispiel 3: 2,6-Bis-o-tolyl-9,10-bis-(2-biphenyl)-anthracen

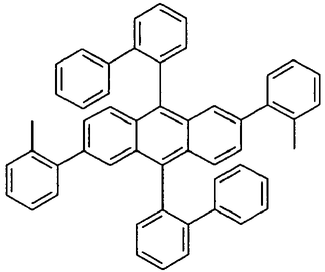

Durchführung analog Beispiel 1 b. Anstelle von 18.0 ml (150 mmol) 2-Bromtoluol werden 25.9 ml (150 mmol) 2-Brombiphenyl verwendet. Umkristallisation aus Chlorbenzol. Sublimation p = 1 x 10⁻⁵ mbar, T = 360 °C. Ausbeute: 27.1 g (41 mmol), 81.7 % d. Th.; Reinheit: 99.9 % n. HPLC. Atropisomerenrein n. ¹H-NMR-Spektroskopie.

### Beispiel 4: 2,6-Bis-o-tolyl-9,10-bis-(2-trimethylsilylphenyl)-anthracen

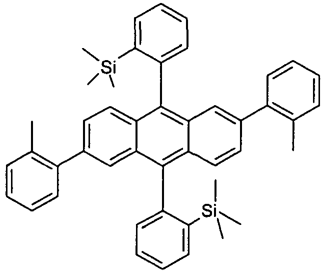

Durchführung analog Beispiel 1b. Anstelle von 18.0 ml (150 mmol) 2-Bromtoluol werden 34.4 g (150 mmol) 2-Trimethylsilyl-brombenzol verwendet. Umkristallisation aus Dioxan. Sublimation p = 1 x 10⁻⁵ mbar, T = 330 °C. Ausbeute: 21.9 g (33 mmol), 66.8 % d. Th.; Reinheit: 99.9 % n. HPLC. Atropisomerenrein n. ¹H-NMR-Spektroskopie.

### Beispiel 5:

Analog zu den Beispielen 1 b, 2, 3, 4 werden folgende Verbindungen dargestellt:

| Bsp. | Bromid | Produkt |
|---|---|---|
| 6 | 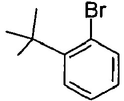 | 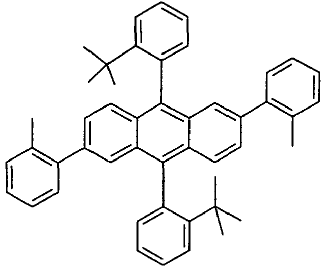 |
| 7 | 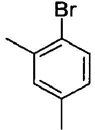 | 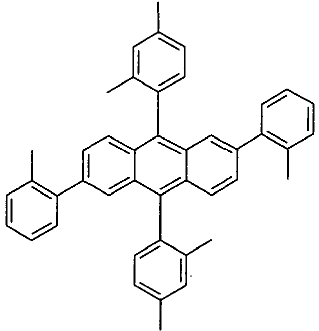 |
| 8 | 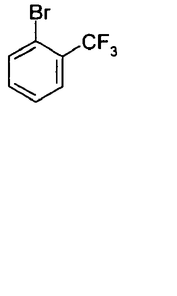 | 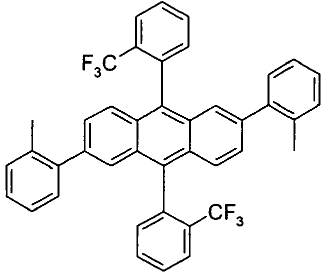 |
| 9 | 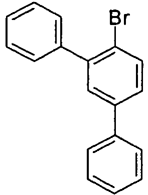 | 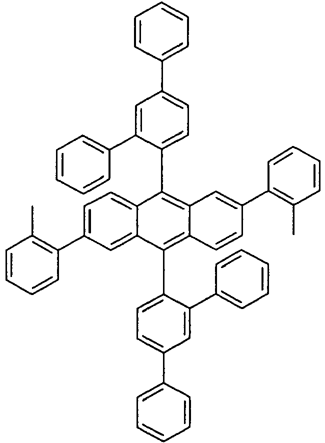 |
| 10 | 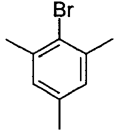 | 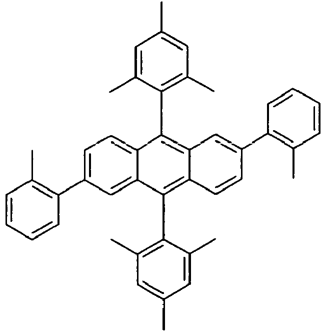 |
| 11 | 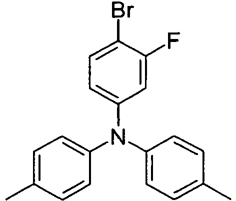 | 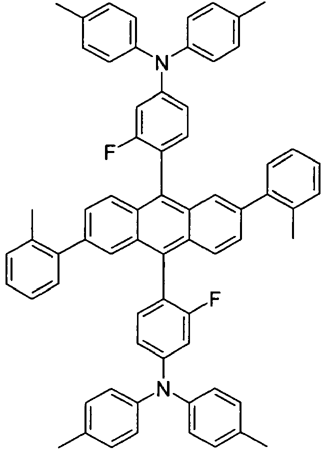 |
| 12 | 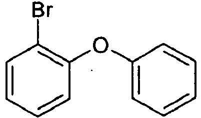 | 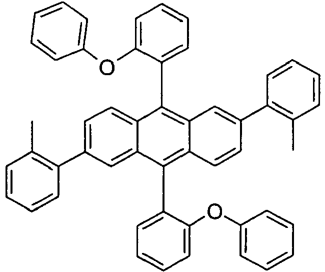 |

### Beispiel 13: 2,6-Bis-naphth-1-yl-9,10-bis-o-tolyl-anthracen

### a) 2,6-Bis-naphth-1-yl-anthrachinon

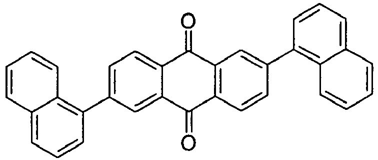

Eine Suspension von 28.7 g (100 mmol) 2,6-Dibrom-anthrachinon, 44.7 g (260 mmol) 1-Naphthylboronsäure, 89.2 g (420 mmol) Kaliumphosphat, 1.8 g (6 mmol) Tri-o-tolylphosphin und 225 mg (1 mmol) Palladium(II)-acetat in einem Gemisch aus 200 ml Dioxan, 400 ml Toluol und 500 ml Wasser wird 16 h unter Rückfluss erhitzt. Nach Erkalten wird der Feststoff abgesaugt, dreimal mit je 100 ml Wasser und dreimal mit je 100 ml Ethanol gewaschen, im Vakuum getrocknet und anschließend zweimal aus Chlorbenzol umkristallisiert. Ausbeute: 41.6 g (90 mmol), 90.3 % d. Th., Reinheit: 99 % n. NMR.

### b) 2,6-Bis-naphth-1-yl-9,10-bis-o-tolyl-anthracen

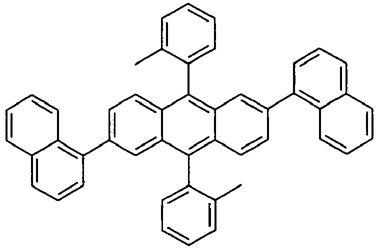

Durchführung analog Beispiel 1b. Umkristallisation aus NMP. Sublimation p = 1 x 10⁻⁵ mbar, T = 375 °C. Ausbeute: 22.2 g (36 mmol), 72.7 % d. Th.; Reinheit: 99.9 % n. HPLC. Mischung aus zwei Atropisomeren n. ¹H-NMR-Spektroskopie.

### Beispiel 14:

Analog zu Beispiel 13 werden folgende Verbindungen dargestellt:

| Bsp. | Bromid | Produkt |
|---|---|---|
| 15 | 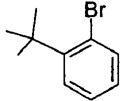 | 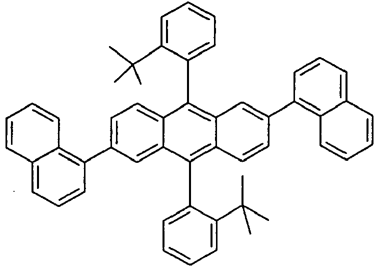 |
| 16 | 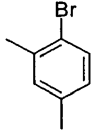 | 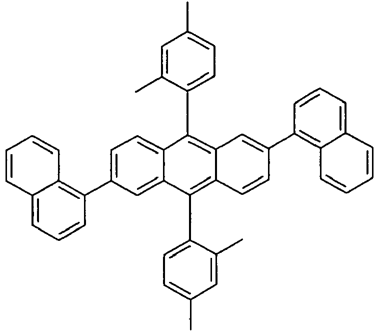 |
| 17 | 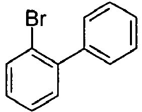 | 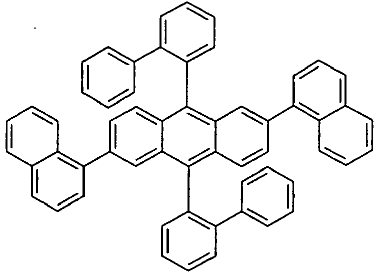 |

### Beispiel 18: 2,6-Bis-(9-(4-methylnaphthyl)anthracen-10-yl)-9,10-bis-o-tolylanthracen

### a) 2,6-Bis-(9-(4-methylnaphthyl)anthracen-10-yl)-anthrachinon

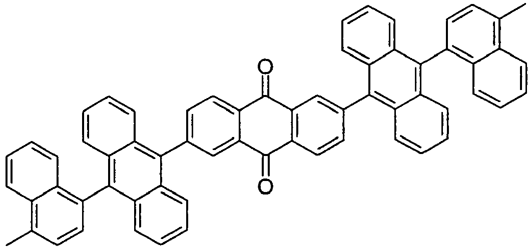

Eine Suspension von 28.7 g (100 mmol) 2,6-Dibrom-anthrachinon, 133.3 g (300 mmol) 10-(4-Methylnaphth-1-yl)anthracen-9-boronsäurepinacolester 96.7 g (600 mmol) Kaliumfluorid und 1.2 g (1 mmol) Tetrakis-triphenyl-phosphino-palladium(0) in einem Gemisch aus 500 ml Ethylenglycoldimethylether, 200 ml Ethanol und 400 ml Wasser wird 36 h unter Rückfluss erhitzt. Nach Erkalten wird der Feststoff abgesaugt, dreimal mit je 100 ml Wasser und dreimal mit je 100 ml Ethanol gewaschen, im Vakuum getrocknet und anschließend zweimal aus o-Dichlorbenzol umkristallisiert. Ausbeute: 66.9 g (79 mmol), 79.5 % d.Th., Reinheit 98 %ig n. NMR.

### b) 2,6-Bis-(9-(4-methylnaphthyl)anthracen-10-yl)-9,10-bis-o-tolyl-anthracen

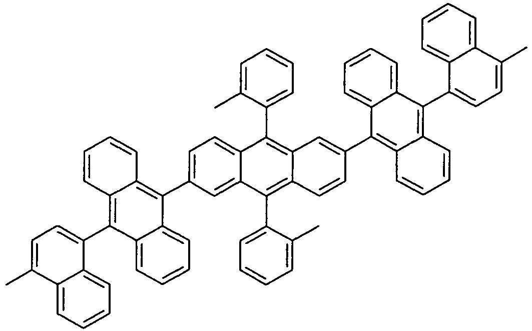

Durchführung analog Beispiel 1b. Anstelle von 19.4 g (50 mmol) 2,6-Bis-o-tolyl-anthrachinon werden 42.1 g (50 mmol) 2,6-Bis-(9-(4-methylnaphthyl)-anthracen-10-yl)-anthrachinon verwendet. Der Reaktionsmischung werden nach Zugabe des 2,6-Bis-(9-(4-methylnaphthyl)anthracen-10-yl)-anthra-chinon 300 ml Toluol zugesetzt. Umkristallisation aus o-Dichlorbenzol. Sublimation p = 1 x 10⁻⁵ mbar, T = 400 °C. Ausbeute: 27.5 g (28 mmol), 55.5 % d. Th.; Reinheit: 99.9 % n. HPLC. Mischung aus zwei Atropisomeren n. ¹H-NMR-Spektroskopie.

### Beispiel 19: 2,6-Bis(p-tolylamino)-9,10-bis-o-tolylanthracen

### a) 2,6-Dibrom-9,10-bis-o-tolylanthracen

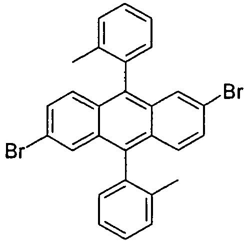

Durchführung analog Beispiel 1 b. Anstelle von 19.4 g (50 mmol) 2,6-Bis-o-tolyl-anthrachinon werden 18.3 g (50 mmol) 2,6-Dibrom-anthrachinon verwendet. Umkristallisation aus Toluol. Ausbeute: 12.3 (24 mmol), 47.6 % d. Th.; Reinheit: 97 % n. NMR.

### b) 2,6-Bis(di-p-tolylamino-phenyl-4-yl)-9,10-bis-o-tolylanthracen

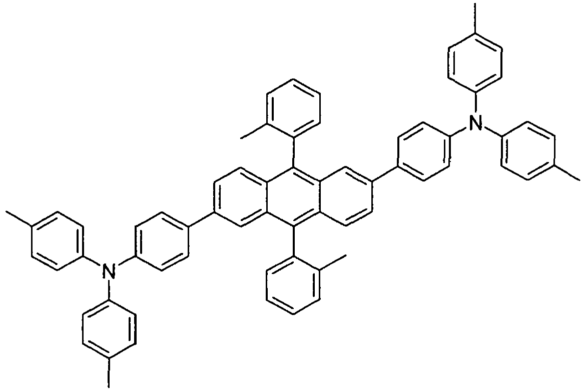

Eine Suspension von 51.6 g (100 mmol) 2,6-Dibrom-9,10-bis-o-tolyl-anthracen, 82.5 g (260 mmol) Di-p-tolyl-amino-phenyl-4-boronsäure, 89.2 g (420 mmol) Kaliumphosphat, 1.8 g (6 mmol) Tri-o-tolylphosphin und 225 mg (1 mmol) Palladium(II)acetat in einem Gemisch aus 200 ml Dioxan, 400 ml Toluol und 500 ml Wasser wird 16 h unter Rückfluss erhitzt. Nach Erkalten wird der Feststoff abgesaugt, dreimal mit je 100 ml Wasser, dreimal mit je 100 ml Ethanol gewaschen, im Vakuum getrocknet, fünfmal aus DMF umkristallisiert und dann im Vakuum (p = 1 x 10⁻⁵ mbar, T = 365 °C) sublimiert. Ausbeute: 68.7 g (76 mmol), 76.2 % d. Th.; Reinheit: 99.9 % n. HPLC.

### Beispiel 20:

Analog zu Beispiel 19 werden folgende Verbindungen dargestellt:

| Bsp. | Amin | Produkt |
|---|---|---|
| 21 | 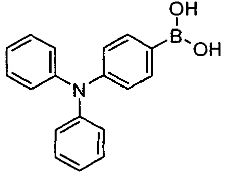 | 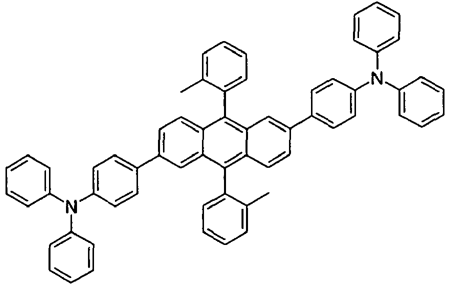 |
| 22 | 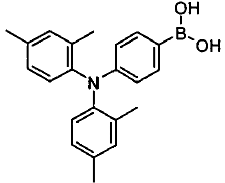 | 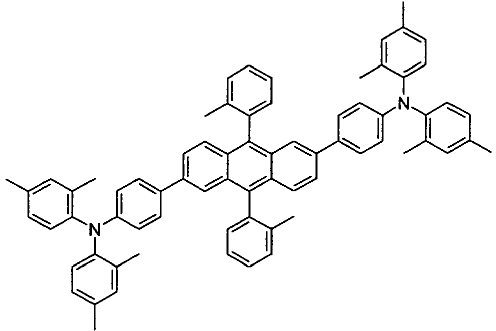 |
| 23 | 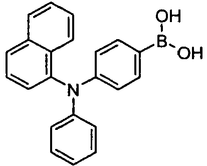 | 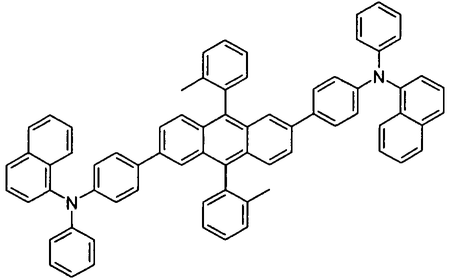 |
| 24 | 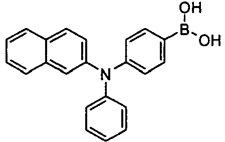 | 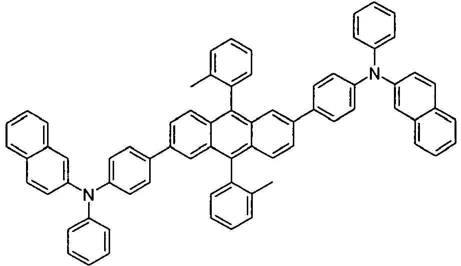 |
| 25 | 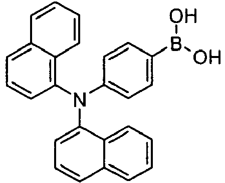 | 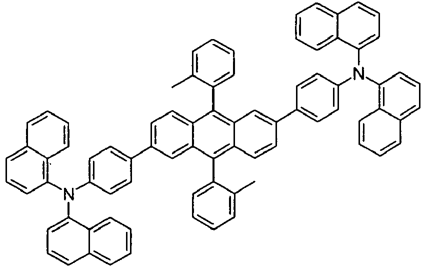 |
| 26 | 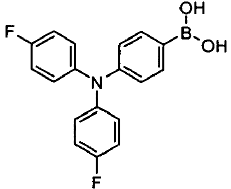 | 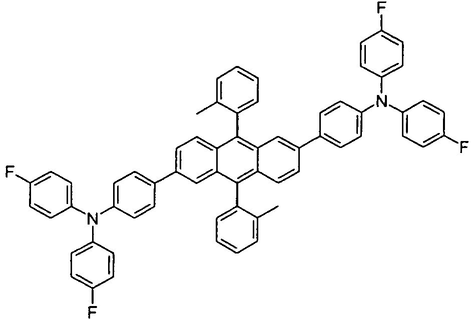 |
| 27 | 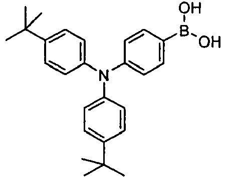 | 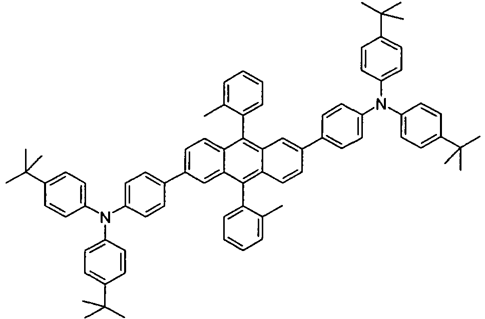 |
| 28 | 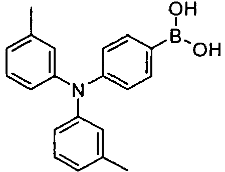 | 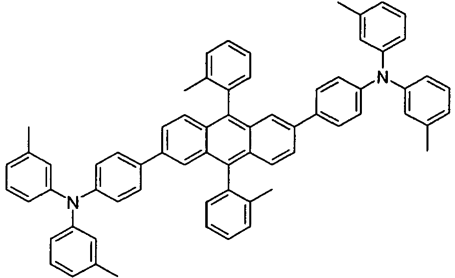 |

### Beispiel 29: 2,6-Bis-naphth-1-yl-9-o-tolyl-10-2-biphenyl-anthracen

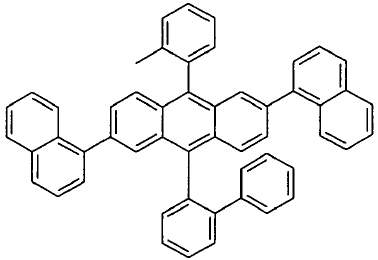

Eine Suspension von 46.1 g (100 mmol) 2,6-Bis-naphth-1-yl-anthrachinon in 500 ml THF wird bei -78 °C unter gutem Rühren tropfenweise mit einer 2-Lithiobiphenyl-Lösung in 200 ml THF, dargestellt aus 11.7 g (50 mmol) 2-Brombiphenyl und 20 ml (50 mmol) 2.5 N n-Buthyllitium bei -78 °C, versetzt und 30 min. nachgerührt. Anschließend wird zu dieser Suspension eine 2-Lithotoluol-Lösung in THF, dargestellt aus 8.7 g (50 mmol) 2-Bromtoluol und 20 ml (50 mmol) 2.5 N n-Buthyllitium bei -78 °C, versetzt und 30 min. nachgerührt. Man lässt die Reaktionsmischung auf Raumtemperatur erwärmen, versetzt mit 30 ml Ethanol und entfernt das Lösemittel im Vakuum. Der Rückstand wird in 300 ml DMF aufgenommen, auf 60 °C erwärmt und unter gutem Rühren portionsweise (Achtung: exotherme Reaktion!) mit 17.7 g (130 mmol) Zinn(II)chlorid versetzt. Anschließend wird noch 2 h bei 60 °C nachgerührt. Nach Erkalten wird mit 500 ml 2.5 N Salzsäure versetzt und vom Feststoff abgesaugt. Der Feststoff wird dreimal mit je 100 ml 2.5 N Salzsäure, dreimal mit je 100 ml Wasser und dreimal mit je 100 ml Ethanol gewaschen, im Vakuum getrocknet, anschließend einmal aus Essigsäure und dreimal aus Dioxan umkristallisiert. Sublimation p = 1 x 10⁻⁵ mbar, T = 345 °C. Ausbeute: 43.1 g (64 mmol), 64.0 % d. Th.; Reinheit: 99.9 % n. HPLC. Mischung aus zwei Atropisomeren n. ¹H-NMR-Spektroskopie.

### Beispiel 30:

Analog zu Beispiel 29 werden folgende Verbindungen dargestellt:

| Bsp. | Bromide | Produkt |
|---|---|---|
| 31 | 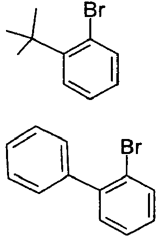 | 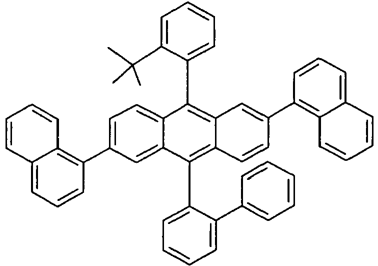 |
| 32 | 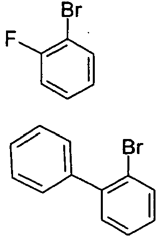 | 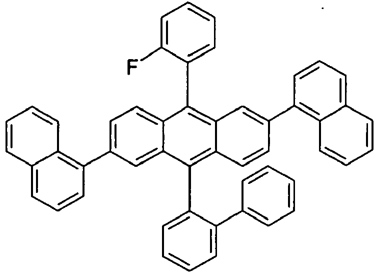 |
| 33 | 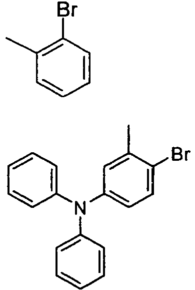 | 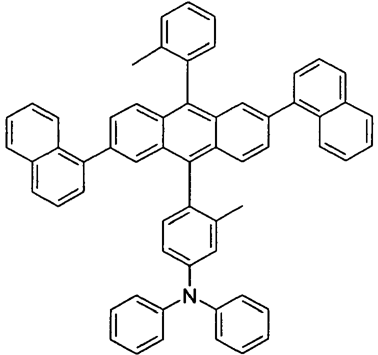 |
| 34 | 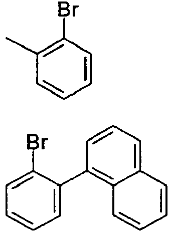 | 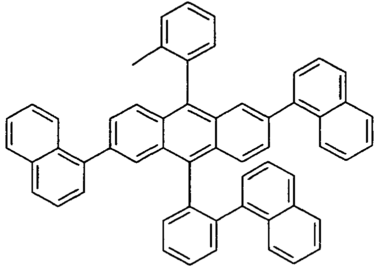 |

### Beispiel 35: Herstellung der OLEDs

Die Herstellung von OLEDs erfolgt nach einem allgemeinen Verfahren gemäß WO 04/058911, das im Einzelfall auf die jeweiligen Gegebenheiten (z. B. Schichtdickenvariation, um optimale Effizienz bzw. Farbe zu erreichen) angepasst wird.

In den folgenden Beispielen 36 bis 48 werden die Ergebnisse verschiedener OLEDs vorgestellt. Glasplatten, die mit strukturiertem ITO (Indium Zinn Oxid) beschichtet sind, bilden die Substrate der OLEDs. Zur verbesserten Prozessierung wird PEDOT (aus Wasser aufgeschleudert; bezogen von H. C. Starck, Goslar, Deutschland; Poly(3,4-ethylendioxy-2,5-thiophen)) direkt auf das Substrat aufgebracht. Die OLEDs bestehen immer aus folgender Schichtenfolge: Substrat / PEDOT 20 nm / Lochinjektionsschicht (HIL1) 20 nm / Lochtransportschicht (HTM1) 20 nm / Emissionsschicht (EML) 30 nm/ Elektronentransportschicht (ETM1) 20 nm und abschließend eine Kathode. Die Materialien bis auf PEDOT werden in einer Vakuumkammer thermisch aufgedampft. Dabei besteht die EML immer aus einem Matrixmaterial (Host) und einem Dotierstoff (Guest oder Dotand), der durch Coverdampfung dem Host beigemischt wird. Die Kathode wird durch eine 1 nm dünne LiF-Schicht und eine darauf abgeschiedene 150 nm Al-Schicht gebildet. Die Tabelle 2 zeigt die chemischen Strukturen der zum Aufbau der OLEDs verwendeten Materialien.

Diese OLEDs werden standardmäßig charakterisiert; hierfür werden die Elektrolumineszenzspektren, die Effizienz (gemessen in cd/A), die Leistungseffizienz (gemessen in lm/W) in Abhängigkeit der Helligkeit, berechnet aus Strom-Spannungs-Helligkeit-Kennlinien (IUL-Kennlinien), und die Lebensdauer bestimmt. Als Lebensdauer wird die Zeit definiert, nach der die Anfangshelligkeit von 1000 cd/m² auf die Hälfte gesunken ist.

In Tabelle 3 sind die Ergebnisse einiger OLEDs (Beispiele 36 bis 48) zusammengefasst. Als Vergleichsbeispiel wird der Host H1 gemäß dem Stand der Technik verwendet.

**Tabelle 2: Verwendete Verbindungen**

| | |
|---|---|
| 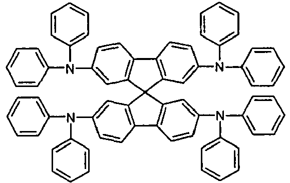 | 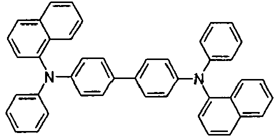 |
| HIL1 | HTM1 |
| 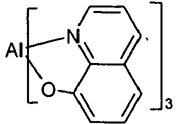 | 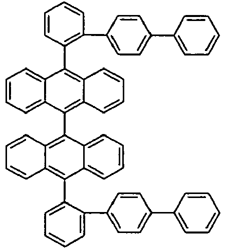 |
| ETM1 | H1 (Vergleich) |
| 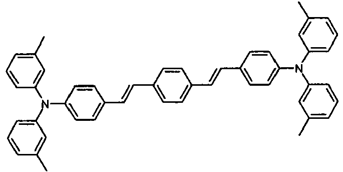 | 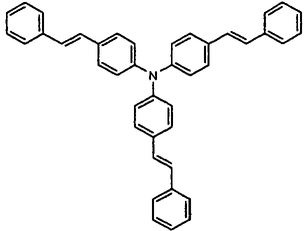 |
| D1 | D2 |

**Tabelle 3: OLED-Ergebnisse**

| **Beispiel** | **EML** | **Max. Effizienz (cd/A)** | **Spannung (V) bei 1000 cd/m²** | **CIE** | **Lebensdauer(h) bei 1000cd/m²** |
|---|---|---|---|---|---|
| 36 (Vergleich) | H1 + 5% D1 | 9.9 | 5.7 | x=0.17 / y=0.33 | 4050 |
| 37 (Vergleich) | H1 + 5% D2 | 3.4 | 6.2 | x=0.15 / y=0.13 | 1200 |
| 38 | Bsp.1 + 5% D1 | 10.5 | 5.5 | x=0.17 / y=0.33 | 6100 |
| 39 | Bsp.1 + 5% D2 | 3.8 | 5.8 | x=0.15 / y=0.14 | 1800 |
| 40 | Bsp.3 + 5% D1 | 12.2 | 5.7 | x=0.17 / y=0.33 | 5800 |
| 41 | Bsp.3 + 5% D2 | 4.2 | 5.9 | x=0.15 / y=0.14 | 1600 |
| 42 | Bsp.9 + 5% D2 | 11.3 | 5.4 | x=0.17 / y=0.32 | 6300 |
| 43 | Bsp.9 + 5% D2 | 3.9 | 5.8 | x=0.15 / y=0.15 | 2200 |
| 44 | Bsp.17 + 5% D3 | 11.5 | 5.5 | x=0.17 / y=0.33 | 7100 |
| 45 | Bsp.17 + 5% D3 | 3.5 | 5.9 | x=0.15 / y=0.14 | 2000 |
| 46 | Bsp.9 + 5% Bsp.11 | 7.8 | 5.3 | x=0.15 / y=0.19 | 4800 |
| 47 | Bsp.9 + 5% Bsp.19 | 8.0 | 5.6 | x=0.15 / y=0.24 | 5300 |
| 48 | Bsp.17 + 7% Bsp.19 | 8.3 | 5.3 | x=0.16 / y=0.26 | 5600 |

## Patentansprüche

1. Verbindungen gemäß Formel (1), 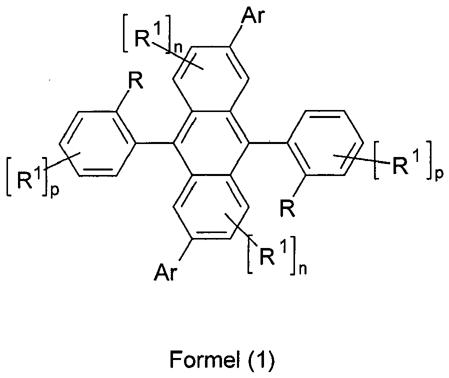 wobei für die verwendeten Symbole und Indizes gilt:
Ar ist bei jedem Auftreten gleich oder verschieden eine Arylgruppe mit 6 bis 16 aromatischen Ringatomen, welche mit einem oder mehreren Resten R¹ substituiert sein kann;
R steht bei jedem Auftreten gleich oder verschieden für Si(R²)₃, N(Ar¹)₂, C(=O)Ar¹, P(=O)(Ar¹)₂, eine geradkettige Alkyl- oder Alkoxygruppe mit 1 bis 10 C-Atomen oder eine verzweigte oder cyclische Alkyl- oder Alkoxygruppe mit 3 bis 10 C-Atomen, die jeweils mit einem oder mehreren Resten R² substituiert sein kann, wobei eine oder mehrere nicht benachbarte CH₂-Gruppen durch -R²C=CR²- oder -O- ersetzt sein können und wobei ein oder mehrere H-Atome durch F ersetzt sein können, oder für eine Arylgruppe mit 5 bis 16 aromatischen Ringatomen, die mit einem oder mehreren Resten R² substituiert sein kann, oder eine Kombination aus zwei, drei oder vier dieser Systeme; dabei können auch benachbarte Substituenten R und R¹ miteinander ein mono- oder polycyclisches, aliphatisches Ringsystem bilden;
R¹ steht bei jedem Auftreten gleich oder verschieden für Si(R²)₃, F, N(Ar¹)₂, eine geradkettige Alkyl- oder Alkoxygruppe mit 1 bis 6 C-Atomen oder eine verzweigte oder cyclische Alkyl- oder Alkoxygruppe mit 3 bis 10 C-Atomen, wobei jeweils ein oder mehrere CH₂-Gruppen durch -R²C=CR²- oder -O- ersetzt sein können und wobei jeweils ein oder mehrere H-Atome durch F ersetzt sein können, oder für eine Arylgruppe mit 5 bis 14 aromatischen Ringatomen, die mit einem oder mehreren Resten R² substituiert sein kann, oder eine Kombination aus zwei oder drei dieser Systeme; dabei können auch zwei oder mehrere Reste R¹ miteinander oder mit einem benachbarten Rest R ein mono- oder polycyclisches, aliphatisches Ringsystem bilden;
Ar¹ ist bei jedem Auftreten gleich oder verschieden ein aromatisches Ringsystem mit 5 bis 30 aromatischen Ringatomen, welches durch einen oder mehrere nichtaromatische Reste R¹ substituiert sein kann; dabei können zwei Reste Ar¹ auch durch eine Einfachbindung oder eine Gruppe O, S, N(R²) oder C(R²)₂ miteinander verbunden sein;
R² ist bei jedem Auftreten gleich oder verschieden H oder ein Kohlenwasserstoffrest mit 1 bis 20 C-Atomen, welcher aliphatisch oder aromatisch oder eine Kombination aus aliphatisch und aromatisch sein kann und welcher auch durch F substituiert sein kann; dabei können auch zwei oder mehrere Reste R² miteinander ein mono- oder polycyclisches, aliphatisches oder aromatisches Ringsystem bilden;
n ist gleich oder verschieden bei jedem Auftreten 0, 1, 2 oder 3;
p ist gleich oder verschieden bei jedem Auftreten 0, 1, 2, 3 oder 4.

2. Verbindungen nach Anspruch 1, **dadurch gekennzeichnet, dass** das Symbol Ar für Phenyl, 1-Naphthyl, 2-Naphthyl, 2-Anthryl, 9-Anthryl, 2-Phenanthrenyl, 3-Phenanthrenyl, 9-Phenanthrenyl, 1-Pyrenyl oder 2-Pyrenyl steht.

3. Verbindungen nach Anspruch 1 oder 2, **dadurch gekennzeichnet, dass** die beiden Substituenten Ar gleich gewählt sind.

4. Verbindungen nach einem oder mehreren der Ansprüche 1 bis 3
gemäß der Formel (2), (3), (4) und (5), 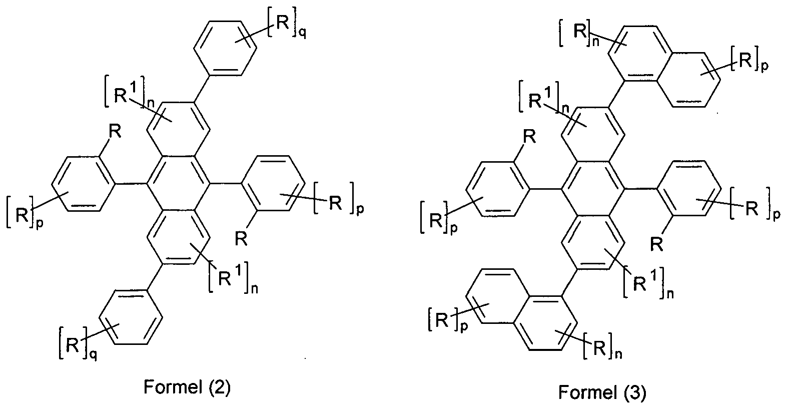 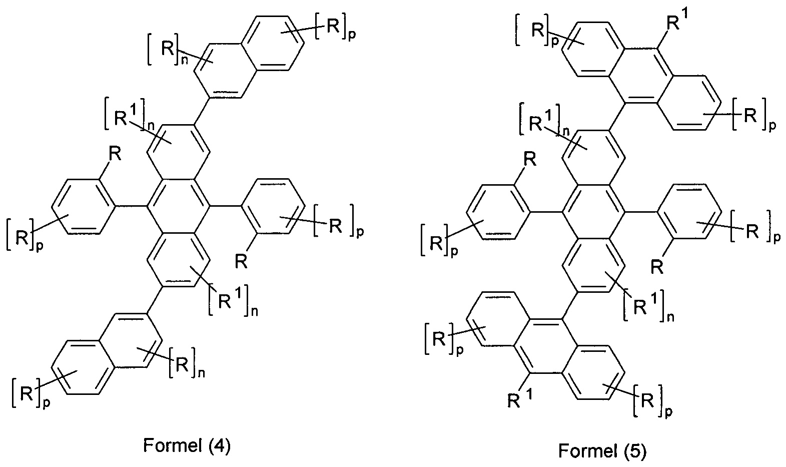 wobei R, R¹, n und p dieselbe Bedeutung haben, wie unter Anspruch 1 beschrieben, und q für 0, 1, 2, 3, 4 oder 5 steht.

5. Verbindungen nach einem oder mehreren der Ansprüche 1 bis 4, **dadurch gekennzeichnet, dass** die beiden Substituenten R gleich gewählt sind.

6. Verbindungen nach einem oder mehreren der Ansprüche 1 bis 5, **dadurch gekennzeichnet, dass** der Index n für 0 oder 1 steht.

7. Verbindungen nach einem oder mehreren der Ansprüche 1 bis 6, **dadurch gekennzeichnet, dass** der Index p für 0, 1 oder 2 steht.

8. Verfahren zur Herstellung von Verbindungen nach einem oder mehreren der Ansprüche 1 bis 7 durch Reaktion eines Anthrachinons, welches in 2,6-Position durch Chlor, Brom, Iod oder einem Sulfonsäurederivat substituiert ist, mit einem Boronsäurederivat der Gruppe Ar unter Palladium-Katalyse, gefolgt von Umsetzung mit einem entsprechenden ortho-substituierten metallorganischen Phenylderivat und Reduktion.

9. Verwendung von Verbindungen nach einem oder mehreren der Ansprüche 1 bis 7 in organischen elektronischen Vorrichtungen, insbesondere in organischen Elektrolumineszenzvorrichtungen.

10. Organische elektronische Vorrichtung, ausgewählt aus der Gruppe bestehend aus organischen Elektrolumineszenzvorrichtungen (OLEDs, PLEDs), organischen Feld-Effekt-Transistoren (O-FETs), organischen Dünnfilmtransistoren (O-TFTs), organischen lichtemittierenden Transistoren (O-LETs), organischen integrierten Schaltungen (O-ICs), organischen Solarzellen (O-SCs), organischen Feld-Quench-Devices (O-FQDs), organischen Photorezeptoren, lichtemittierenden elektrochemischen Zellen (LECs) und organische Laserdioden (O-Laser), insbesondere organischen Elektrolumineszenzvorrichtungen, enthaltend mindestens eine Verbindung nach einem oder mehreren der Ansprüche 1 bis 7.

11. Organische Elektrolumineszenzvorrichtungen nach Anspruch 10, enthaltend Anode, Kathode und mindestens eine emittierende Schicht und gegebenenfalls weitere Schichten, ausgewählt aus Lochinjektionsschicht, Lochtransportschicht, Lochblockierschicht, Elektronentransportschicht, Elektroneninjektionsschicht und/oder Charge-Generation Layer.

12. Organische Elektrolumineszenzvorrichtung nach Anspruch 11, **dadurch gekennzeichnet, dass** eine Verbindung nach einem oder mehreren der Ansprüche 1 bis 7 als Hostmaterial für fluoreszierende Emitter und/oder als Elektronentransportmaterial und/oder als Lochblockiermaterial verwendet wird.

13. Organische Elektrolumineszenzvorrichtung nach Anspruch 12, **dadurch gekennzeichnet, dass** die Dotanden ausgewählt sind aus der Klasse der aromatischen Anthracenamine, der aromatischen Anthracendiamine, der aromatischen Pyrenamine, der aromatischen Pyrendiamine, der Monostyrylamine, der Distyrylamine, der Tristyrylamine, der Tetrastyrylamine, der Styrylphosphine, der Styrylether und der Arylamine.

14. Organische Elektrolumineszenzvorrichtung nach einem oder mehreren der Ansprüche 11 bis 13, **dadurch gekennzeichnet, dass** eine Verbindung nach einem oder mehreren der Ansprüche 1 bis7 als emittierende Verbindung in einer emittierenden Schicht und/oder als Lochtransportmaterial, insbesondere in einer Lochtransportschicht oder einer Lochinjektionsschicht verwendet wird, insbesondere wenn das Symbol R für eine Gruppe N(Ar¹)₂ steht und/oder ein Substituent R¹ für eine Gruppe N(Ar¹)₂ steht.

## Claims

1. Compounds of the formula (1), 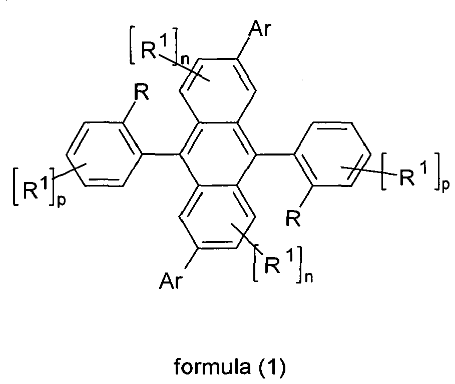 where the following applies to the symbols and indices used:
Ar is on each occurrence, identically or differently, an aryl group having 6 to 16 aromatic ring atoms, which may be substituted by one or more radicals R¹;
R stands on each occurrence, identically or differently, for Si(R²)₃, N(Ar¹)₂, C(=O)Ar¹, P(=O)(Ar¹)₂, a straight-chain alkyl or alkoxy group having 1 to 10 C atoms or a branched or cyclic alkyl or alkoxy group having 3 to 10 C atoms, each of which may be substituted by one or more radicals R², where one or more non-adjacent CH₂ groups may be replaced by -R²C=CR²- or -O- and where one or more H atoms may be replaced by F, or for an aryl group having 5 to 16 aromatic ring atoms, which may be substituted by one or more radicals R², or a combination of two, three or four of these systems; adjacent substituents R and R¹ here may also form a mono- or polycyclic, aliphatic ring system with one another;
R¹ stands on each occurrence, identically or differently, for Si(R²)₃, F, N(Ar¹)₂, a straight-chain alkyl or alkoxy group having 1 to 6 C atoms or a branched or cyclic alkyl or alkoxy group having 3 to 10 C atoms, where in each case one or more CH₂ groups may be replaced by -R²C=CR²- or -O- and where in each case one or more H atoms may be replaced by F, or for an aryl group having 5 to 14 aromatic ring atoms, which may be substituted by one or more radicals R², or a combination of two or three of these systems; two or more radicals R¹ here may also form a mono- or polycyclic, aliphatic ring system with one another or with an adjacent radical R;
Ar¹ is on each occurrence, identically or differently, an aromatic ring system having 5 to 30 aromatic ring atoms, which may be substituted by one or more non-aromatic radicals R¹; two radicals Ar¹ here may also be connected to one another by a single bond or a group O, S, N(R²) or C(R²)₂;
R² is on each occurrence, identically or differently, H or a hydrocarbon radical having 1 to 20 C atoms, which may be aliphatic or aromatic or a combination of aliphatic and aromatic and which may also be substituted by F; two or more radicals R² here may also form a mono- or polycyclic, aliphatic or aromatic ring system with one another;
n is, identically or differently on each occurrence, 0, 1, 2 or 3;
p is, identically or differently on each occurrence, 0, 1, 2, 3 or 4.

2. Compounds according to Claim 1, **characterised in that** the symbol Ar stands for phenyl, 1-naphthyl, 2-naphthyl, 2-anthryl, 9-anthryl, 2-phenanthrenyl, 3-phenanthrenyl, 9-phenanthrenyl, 1-pyrenyl or 2-pyrenyl.

3. Compounds according to Claim 1 or 2, **characterised in that** the two substituents Ar are selected identically.

4. Compounds according to one or more of Claims 1 to 3 of the formulae (2), (3), (4) and (5), 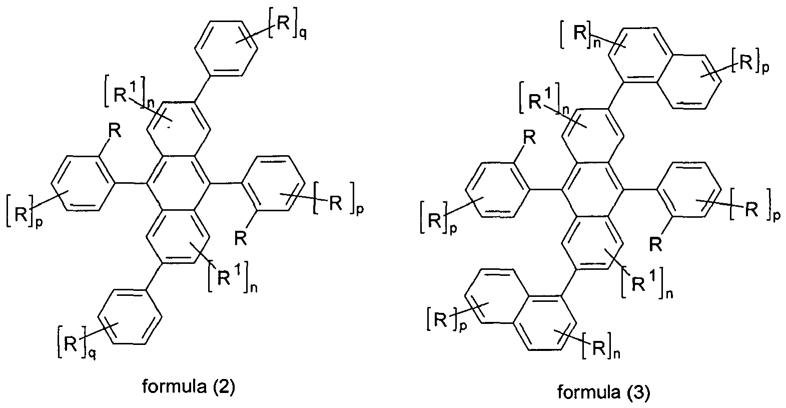 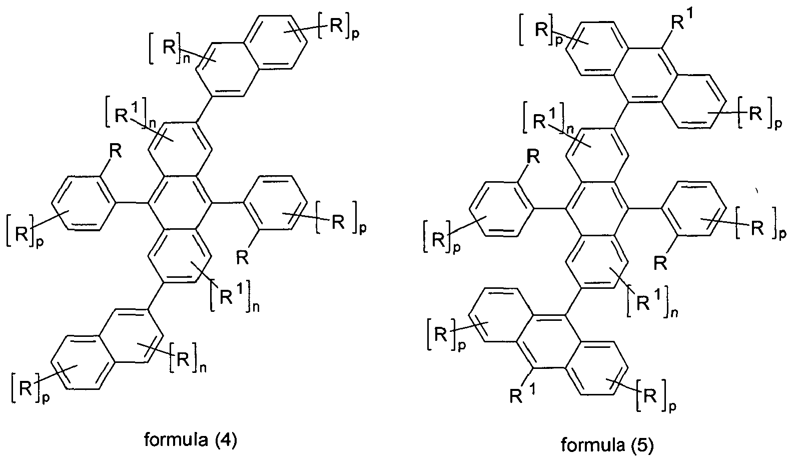 where R, R¹, n and p have the same meaning as described under Claim 1 and q stands for 0, 1, 2, 3, 4 or 5.

5. Compounds according to one or more of Claims 1 to 4, **characterised in that** the two substituents R are selected identically.

6. Compounds according to one or more of Claims 1 to 5, **characterised in that** the index n stands for 0 or 1.

7. Compounds according to one or more of Claims 1 to 6, **characterised in that** the index p stands for 0, 1 or 2.

8. Process for the preparation of compounds according to one or more of Claims 1 to 7 by reaction of an anthraquinone which is substituted in the 2,6-position by chlorine, bromine, iodine or a sulfonic acid derivative with a boronic acid derivative of the group Ar with palladium catalysis, followed by reaction with a corresponding ortho-substituted organometallic phenyl derivative and reduction.

9. Use of compounds according to one or more of Claims 1 to 7 in organic electronic devices, in particular in organic electroluminescent devices.

10. Organic electronic device selected from the group consisting of organic electroluminescent devices (OLEDs, PLEDs), organic field-effect transistors (O-FETs), organic thin-film transistors (O-TFTs), organic light-emitting transistors (O-LETs), organic integrated circuits (O-ICs), organic solar cells (O-SCs), organic field-quench devices (O-FQDs), organic photoreceptors, light-emitting electrochemical cells (LECs) and organic laser diodes (O-lasers), in particular organic electroluminescent devices, comprising at least one compound according to one or more of Claims 1 to 7.

11. Organic electroluminescent device according to Claim 10 comprising anode, cathode and at least one emitting layer and optionally further layers selected from hole-injection layer, hole-transport layer, hole-blocking layer, electron-transport layer, electron-injection layer and/or charge-generation layer.

12. Organic electroluminescent device according to Claim 11, **characterised in that** a compound according to one or more of Claims 1 to 7 is used as host material for fluorescent emitters and/or as electron-transport material and/or as hole-blocking material.

13. Organic electroluminescent device according to Claim 12, **characterised in that** the dopants are selected from the class of the aromatic anthracenamines, the aromatic anthracenediamines, the aromatic pyrenamines, the aromatic pyrenediamines, the monostyrylamines, the distyrylamines, the tristyrylamines, the tetrastyrylamines, the styrylphosphines, the styryl ethers and the arylamines.

14. Organic electroluminescent device according to one or more of Claims 11 to 13, **characterised in that** a compound according to one or more of Claims 1 to 7 is used as emitting compound in an emitting layer and/or as hole-transport material, in particular in a hole-transport layer or a hole-injection layer, in particular if the symbol R stands for a group N(Ar¹)₂ and/or a substituent R¹ stands for a group N(Ar¹)₂.

## Revendications

1. Composés de la formule (1) : 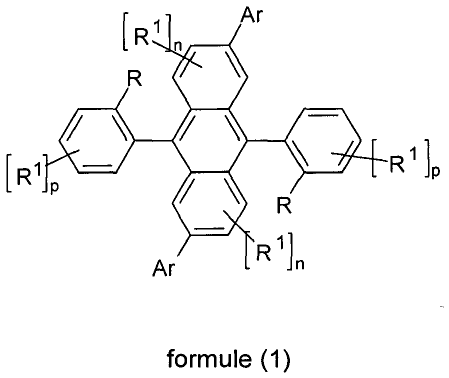 dans laquelle ce qui suit s'applique aux symboles et indices utilisés :
Ar est, pour chaque occurrence, de manière identique ou différente, un groupe aryle comportant de 6 à 16 atomes de cycle aromatique, lequel peut être substitué par un radical ou plusieurs radicaux R¹ ;
R représente, pour chaque occurrence, de manière identique ou différente, Si(R²)₃, N(Ar¹)₂, C(=O)Ar¹, P(=O)(Ar¹)₂, un groupe alkyle ou alcoxy en chaîne droite comportant de 1 à 10 atome(s) de C ou un groupe alkyle ou alcoxy ramifié ou cyclique comportant de 3 à 10 atomes de C, dont chacun peut être substitué par un radical ou plusieurs radicaux R², où un ou plusieurs groupes CH₂ non adjacents peuvent être remplacés par -R²C=CR²- ou -O- et où un ou plusieurs atome(s) de H peut/peuvent être remplacé(s) par F, ou un groupe aryle comportant de 5 à 16 atomes de cycle aromatique, lequel peut être substitué par un radical ou plusieurs radicaux R², ou une combinaison de deux, trois ou quatre de ces systèmes ; des substituants R et R¹ adjacents peuvent ici également former un système de cycle aliphatique monocyclique ou polycyclique l'un avec l'autre ou les uns avec les autres ;
R¹ représente, pour chaque occurrence, de manière identique ou différente, Si(R²)₃, F, N(Ar¹)₂, un groupe alkyle ou alcoxy en chaîne droite comportant de 1 à 6 atome(s) de C ou un groupe alkyle ou alcoxy ramifié ou cyclique comportant de 3 à 10 atomes de C, où, dans chaque cas, un ou plusieurs groupe(s) CH₂ peut/peuvent être remplacé(s) par -R²C=CR²- ou -O- et où, dans chaque cas, un ou plusieurs atome(s) de H peut/peuvent être remplacé(s) par F, ou un groupe aryle comportant de 5 à 14 atomes de cycle aromatique, lequel peut être substitué par un radical ou plusieurs radicaux R², ou une combinaison de deux ou trois de ces systèmes ; deux radicaux R¹ ou plus peuvent ici également former un système de cycle aliphatique monocyclique ou polycyclique l'un avec l'autre ou les uns avec les autres ou avec un radical R adjacent ;
Ar¹ est, pour chaque occurrence, de manière identique ou différente, un système de cycle aromatique comportant de 5 à 30 atomes de cycle aromatique, lequel peut être substitué par un radical ou plusieurs radicaux R¹ non aromatique(s) ; deux radicaux Ar¹ peuvent ici également être connectés l'un à l'autre par une liaison simple ou un groupe O, S, N(R²) ou C(R²)₂ ;
R² est, pour chaque occurrence, de manière identique ou différente, H ou un radical hydrocarbone comportant de 1 à 20 atome(s) de C, lequel peut être aliphatique ou aromatique ou une combinaison de aliphatique et aromatique et lequel peut également être substitué par F ; deux radicaux R² ou plus peuvent ici également former un système de cycle aliphatique ou aromatique monocyclique ou polycyclique l'un avec l'autre ou les uns avec les autres ;
n est, de manière identique ou différente pour chaque occurrence, 0, 1, 2 ou 3 ;
p est, de manière identique ou différente pour chaque occurrence, 0, 1, 2, 3 ou 4.

2. Composés selon la revendication 1, **caractérisés en ce que** le symbole Ar représente phényle, 1-naphtyle, 2-naphtyle, 2-anthryle, 9-anthryle, 2-phénanthrényle, 3-phénanthrényle, 9-phénanthrényle, 1-pyrényle ou 2-pyrényle.

3. Composés selon la revendication 1 ou 2, **caractérisés en ce que** les deux substituants Ar sont choisis de manière à être identiques.

4. Composés selon une ou plusieurs des revendications 1 à 3 des formules (2), (3), (4) et (5) : 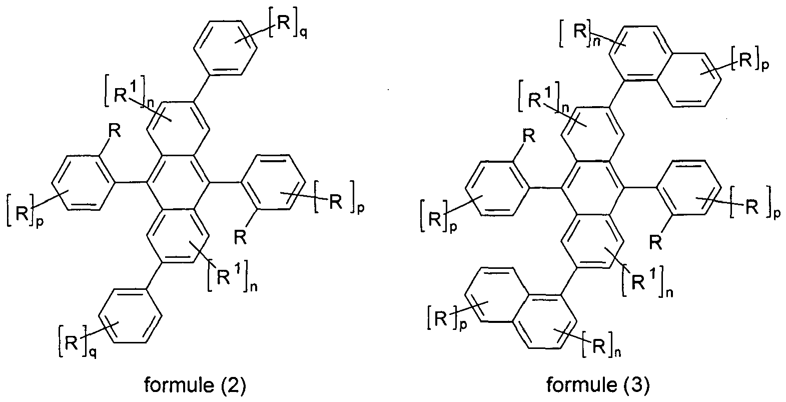 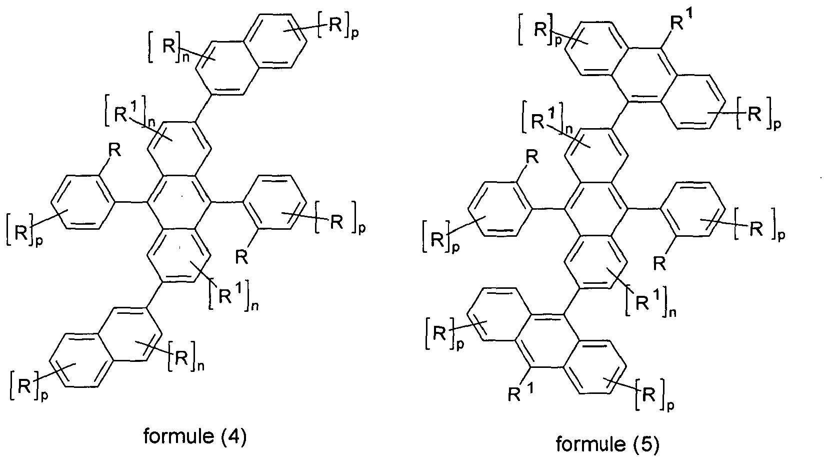 dans lesquelles R, R¹, n et p présentent la même signification que décrit selon la revendication 1 et q représente 0, 1, 2, 3, 4 ou 5.

5. Composés selon une ou plusieurs des revendications 1 à 4, **caractérisés en ce que** les deux substituants R sont choisis de manière à être identiques.

6. Composés selon une ou plusieurs des revendications 1 à 5, **caractérisés en ce que** l'indice n représente 0 ou 1.

7. Composés selon une ou plusieurs des revendications 1 à 6, **caractérisés en ce que** l'indice p représente 0, 1 ou 2.

8. Procédé pour la préparation de composés selon une ou plusieurs des revendications 1 à 7 au moyen de la réaction d'un anthraquinone qui est substitué à la position 2,6 par chlore, brome, iode ou un dérivé d'acide sulfonique avec un dérivé d'acide boronique du groupe Ar moyennant une catalyse par palladium, réaction suivie par une réaction avec un dérivé de phényle organométallique ortho-substitué correspondant et par une réduction.

9. Utilisation de composés selon une ou plusieurs des revendications 1 à 7 dans des dispositifs électroniques organiques, en particulier dans des dispositifs électroluminescents organiques.

10. Dispositif électronique organique choisi parmi le groupe constitué par des dispositifs électroluminescents organiques (OLED, PLED), des transistors à effet de champ organiques (O-FET), des transistors à film mince organiques (O-TFT), des transistors à émission de lumière organiques (O-LET), des circuits intégrés organiques (O-IC), des cellules solaires organiques (O-SC), des dispositifs à extinction de champ organiques (O-FQD), des photorécepteurs organiques, des cellules électrochimiques à émission de lumière (LEC) et des diodes laser organiques (O-laser), en particulier des dispositifs électroluminescents organiques, comprenant au moins un composé selon une ou plusieurs des revendications 1 à 7.

11. Dispositif électroluminescent organique selon la revendication 10 comprenant une anode, une cathode et au moins une couche d'émission et en option, d'autres couches choisies parmi une couche d'injection de trous, une couche de transport de trous, une couche de blocage de trous, une couche de transport d'électrons, une couche d'injection d'électrons et/ou une couche de génération de charges.

12. Dispositif électroluminescent organique selon la revendication 11, **caractérisé en ce qu'**un composé selon une ou plusieurs des revendications 1 à 7 est utilisé en tant que matériau hôte pour des émetteurs fluorescents et/ou en tant que matériau de transport d'électrons et/ou en tant que matériau de blocage de trous.

13. Dispositif électroluminescent organique selon la revendication 12, **caractérisé en ce que** les dopants sont choisis parmi la classe des anthracénamines aromatiques, des anthracénédiamines aromatiques, des pyrénamines aromatiques, des pyrénédiamines aromatiques, des monostyrylamines, des distyrylamines, des tristyrylamines, des tétrastyrylamines, des styrylphosphines, des styryl éthers et des arylamines.

14. Dispositif électroluminescent organique selon une ou plusieurs des revendications 11 à 13, **caractérisé en ce qu'**un composé selon une ou plusieurs des revendications 1 à 7 est utilisé en tant que composé d'émission dans une couche d'émission et/ou en tant que matériau de transport de trous, en particulier dans une couche de transport de trous ou une couche d'injection de trous, en particulier si le symbole R représente un groupe N(Ar¹)₂ et/ou un substituant R¹ représente un groupe N(Ar¹)₂.
